# EUROPEAN PATENT SPECIFICATION

(11) **EP 3 054 965 B1**
(45) Date of publication and mention of the grant of the patent: **14.04.2021**
(21) Application number: 14852988.6
(22) Date of filing: 07.10.2014
(51) Int. Cl.: A61K 38/00, A61K 48/00, A61K 39/395, A61P 27/02

(54) **ANTI-FACTOR D ANTIBODY FOR USE IN PREVENTING OR REDUCING RETINAL DETACHMENT**
ANTI-FAKTOR D ANTIKÖRPER ZUR BEHANDLUNG ODER REDUZIERUNG DER NETZHAUTABLÖSUNG
ANTICORPS ANTI-FACTEUR D POUR LE TRAITEMENT OU LA RÉDUCTION DU DÉCOLLEMENT DE RÉTINE

(30) Priority: 07.10.2013 US 201361887757 P
(43) Date of publication of application: 17.08.2016
(73) Proprietor: Massachusetts Eye & Ear Infirmary, Boston, MA 02214 (US)
(72) Inventor: CONNOR, Kip, M., West Newton, MA 02465 (US)
(74) Representative: Lahrtz, Fritz
(86) International application number: PCT/US2014/059550
(87) International publication number: WO 2015/054298

(56) References cited:
- WO-A1-2008/147883
- WO-A2-2007/056227
- WO-A2-2007/056227
- WO-A2-2007/103549
- WO-A2-2007/103549
- US-A1- 2008 269 318
- US-A1- 2009 269 338
- US-A1- 2010 291 106
- KOCSIS ET AL.: 'Selective Inhibition of the Lectin Pathway of Complement with Phage Display Selected Peptides against Mannose-Binding Lectin-Associated Serine Protease (MASP)-1 and - 2: Significant Contribution of MASP-1 to Lectin Pathway Activation' THE JOURNAL OF IMMUNOLOGY vol. 185, 2010, pages 4169 - 4178, XP007915332

## Description

### FIELD OF THE INVENTION

This invention relates generally to the field of ophthalmology.

### BACKGROUND OF THE INVENTION

Retinal detachment (RD) occurs in various retinal disorders, including retinal tears, age-related macular degeneration and diabetic retinopathy, as well as a number of clinical manifestations such as tractional, rhegmatogenous and exudative RD (Zacks, D. N. et al., Invest Ophthalmol Vis Sci, 2006, 47:1691-1695). In patients with sustained RD, progressive visual decline due to photoreceptor cell death is common and leads to a significant decrease in visual acuity (Day S. et al., Am J Ophthalmol, 2010, 150:338-345; and Rowe J.A. et al., Opthalmology, 1999, 106:154-159). While numerous pathological changes occur in the detached retina, studies in human patient samples and in animal models have shown that photoreceptor cell death is induced as early as 12 hours and peaks at around 2-3 days after RD (Yu, J. et al., Invest Ophthalmol Vis Sci, 2012, 53:8146-8153). Untreated retinal detachment results in permanent vision loss, and frequently results in blindness.

Currently, the only treatment available for retinal detachment is to reattach the retina by surgical procedures. However, surgery is extremely invasive and fraught with risks such as infection, bleeding, increases in intraocular pressure, and cataract. Moreover, surgery is not always effective for reattaching the retina and/or restoring normal vision. Many patients fail to recover any lost vision. Thus, there exists an urgent need for alternative treatments for retinal detachment, for preserving vision and/or preventing photoreceptor cell death associated with retinal detachment.

### SUMMARY OF THE INVENTION

The invention is based on the surprising discovery that photoreceptor degeneration correlates with increased activity of components of the alternative complement pathway. Furthermore, ablation of alternative complement pathway components protects photoreceptor cells from cell death.

Accordingly, compositions for preserving vision, reducing vision loss, and/or inhibiting or reducing photoreceptor cell death in a subject comprise an agent that inhibits or reduces complement pathway activity are described herein. The complement pathway is the alternative complement pathway or the lectin complement pathway. The agent comprises a small molecule, a polynucleotide, a polypeptide, an antibody or an antibody fragment with means to inhibit or reduce the transcription, transcript stability, modification, localization, secretion, or function of a polynucleotide or polypeptide encoding a component of the alternative or lectin complement pathway. For example, the agent comprises a serine protease inhibitor, a soluble form of a complement receptor, a humanized monoclonal antibody or antibody fragment, a complement component inhibitor, a nucleic acid expression vector encoding an anti-complement agent, a modified complement receptor or an anaphylatoxin receptor antagonist.

Preferably, the agent inhibits or reduces the activity of at least one component of the complement pathway, e.g., the agent inhibits binding of one component to another component of the pathway. Preferably, the complement pathway is the alternative complement pathway. Alternatively, the complement pathway is the lectin complement pathway. The agent inhibits or reduces the activity of at least one component of the alternative or lectin pathway complement pathway. Components of the alternative complement pathway include factor B (Fb), C3, properdin (Factor p), factor Ba, factor Bb, factor D, C2, C2a, C3, C3a, C5, C5a, C6, C7, C8, C9, and C5b-9. For example, the inhibitory agent is specific for or binds to a component of the alternate complement pathway as described above. Components of the lectin complement pathway include MASP-1, MASP-2, MASP-3, Map19, Map44, C4, C4a, C4b, C2, C2a and C2b. In a preferred embodiment, the agent specifically binds to the complement pathway component to modulate the transcription, transcript stability, modification, localization, secretion, or function of the component.

For example, the agent that inhibits or reduces the activity of at least one component of the complement pathway is an antibody or an antibody fragment. The antibody or antibody fragment specifically binds to an alternative complement component, such as factor B, C3, properdin (Factor p), factor Ba, factor Bb, factor D, C2, C2a, C3, C3a, C3b C5, C5a, C5b, C6, C7, C8, C9, or C5b-9. The antibody or antibody fragment specifically binds to a lectin complement component, such as MASP-1, MASP-2, MASP-3, Map19, Map44, C4, C4a, C4b, C2, C2a and C2b. The antibody is a monoclonal antibody. The antibody fragment is a Fab fragment, a Fab' fragment, a F(ab')2 fragment, or an ScFv fragment. The antibody is a chimeric antibody. The antibody or antibody fragment is humanized. Preferably, the antibody or fragment thereof is soluble and binds to or inhibits a component or factor of the human alternative complement pathway.

Examples of agents that inhibit or reduce the activity of the complement pathway include, but are not limited to, cinryze, berinert, rhucin, eculizumab, pexelizumab, ofatumumab, TNX-234, compstatin/POT-4, PMX-53, rhMBL, human CD55, BCX-1470, C1-INH, SCR1/TP10, CAB-2/MLN-2222, mirococept, sCR1-sLe^{x}/TP-20, TNX-558, TA106, Neutrazumab, anti-properdin, HuMax-CD38, ARC1905, JPE-1375, and JSM-7717. Examples of agents that inhibit or reduce the activity of the lectin pathway include, but are not limited to, C1-inh, antithrombin, sunflower MASP inhibitors SFMI-1 or SFMI-2, or SMGI inhibitors SGMI-1 or SGMI-2. Some preferred agents that inhibit the complement pathway are disclosed in US Publication 2013/0149373, WO 2013/093762, WO 2010/136311, WO 2009/056631. Other agents are disclosed in Wagner, E. et al., Nature Rev, 2010, 9:43-56, Mucke, H. Et al., IDrugs, 2010, 13(1): 30-37, Ma, K. N. et al., Invest Ophthalmol Vis Sci, 2010, 51(12): 6776-6783, and Ricklin D. et al, Nat Biotech, 2007, 25(11):1265-1275.

The compositions and methods described herein are also useful for inhibiting or reducing photoreceptor cell death by inhibiting or reducing complement pathway activity. Photoreceptor cells, together with photosensitive retinal ganglion cells (RGCs) are responsible for converting light into electric signals. Photoreceptor cell degeneration plays a significant role in retinal detachment and subsequent loss of vision. Thus, prevention or reduction of photoreceptor cell death alleviates or prevents permanent or significant vision loss. Untreated retinal detachment or sustained photoreceptor cell death causes loss and death of retinal ganglion cells.

Photoreceptor cells are primarily distinguished from other retinal cells by their morphology. Photoreceptor cells are divided into rod and cone cells, as defined by their morphology and functional photopigments used. They are localized in the outer part of the retina; their nuclei are found in the outer nuclear layer (ONL) while their-outer segments are oriented toward the retinal pigment epithelium (RPE). Structurally, cone cells are somewhat shorter than rods, but wider and tapered, having a cone-like shape at their outer segment where a pigment filters incoming light. Rod cells are longer than cone cells and leaner, and the pigment on the outer side toward the RPE. Unique extracellular markers have also been identified for photoreceptors. Examples of such markers are Cacna2d4, Kcnv2, Pcdh21, recoverin, Rho4D2, and transducin. Antibodies that specifically bind to a photoreceptor-specific marker can be used to identify photoreceptors.

Photoreceptor cell death can be measured by methods known in the art. For example, immunohistochemical analysis can be used by staining with apoptotic or cell death markers known in the art. The examples described herein demonstrate TUNEL-staining of retinal cross-sections to identify cell death, and correlated to cell morphology to identify photoreceptor-specific cell death.

Spectral domain optical coherence tomography (SD-OCT) is also used for detailed and non-invasive evaluation of the retinal architecture in vivo. SD-OCT accurately reflects retinal morphological changes that occur during retinal disease progression, including retinal detachment.

Alternatively, photoreceptor cell death can be measured by assessing photoreceptor cell function. Loss in photoreceptor cell function indicates loss or death of photoreceptor cells. Electroretinography (ERG) analysis is a method known in the art for assessing photoreceptor function and neural responses. Advantages of this technique include non-invasiveness, and objective evaluation of retinal function on a layer-by-layer basis. In brief, the flash ERG is assessed in a dark adapted eye. The initial α-wave (initial negative deflection) is primarily derived from photoreceptors where the second half of the α-wave is a combination of photoreceptors, bipolar, amacrine, and muller cells. The b-wave (positive deflection) originates in retinal cells that are post-synaptic to the photoreceptors and are used as a readout for photoreceptor function.

Other methods for measuring photoreceptor function include standard eye examinations that are known in the art. For example, a Snellen chart, or variations thereof, containing letters and/or numbers is used to determine visual acuity. Decreased ability to distinguish and recognize the letters and/or numbers indicates loss of photoreceptor function or visual acuity. An increased ability to distinguish and recognize the letters and/or numbers after treatment indicates efficaciousness of the treatment.

The compositions and methods described herein are useful for the preservation of vision can be measured by methods known in the art. For example, preservation of vision can be measured by assessing retinal cell activity (*i.e.,* photoreceptor cell activity) by electroretinography, visual acuity by eye chart tests, and peripheral vision by visual field tests, as described herein.

The subject is a mammal in need of such treatment, e.g., a subject that has been diagnosed with an ocular disorder associated with complement-mediated retinal cell death. For example, the subject has been diagnosed with retinal detachment or a predisposition thereto or has been identified as having experienced a head injury such as traumatic brain injury (TBI). The mammal is, e.g., a human, a primate, a mouse, a rat, a dog, a cat, a horse, as well as livestock or animals grown for food consumption, e.g., cattle, sheep, pigs, chickens, and goats. For example, the mammal is a performance mammal, such as a racehorse or racedog (e.g., greyhound). Preferably, the mammal is a human. In preferred embodiments, the subject does not comprise geographic atrophy (GA), e.g., the subject has not been diagnosed with geographic atrophy of the eye.

Subjects at risk for retinal detachment are individuals that have, for example, nearsightedness (or severe myopia), previous cataract surgery, severe trauma, previous retinal detachment in either eye, family history of retinal detachment, macular degeneration (*i.e.,* wet macular degeneration), diabetic retinopathy (*i.e.,* proliferative diabetic retinopathy), retinopathy of prematurity, eclampsia, homocysteinuria, malignant hypertension, inflammatory conditions (*i.e.,* uveitis or scleritis), glaucoma, retinoblastoma, metastatic cancer that spreads to the eye, choroidal melanoma, haemangioma, Stickler syndrome, Von Hippel-Lindau disease, AIDS, and smoking. Other subjects at risk for retinal detachment are individuals that engage in activities with increased risk for trauma or injury to or in the proximity of the eye. Examples of such subjects include, but are not limited to, boxers, wrestlers, military personnel, young males.

A subject that is suffering from retinal detachment is identified by diagnosis by a physician or clinician using methods known in the art, or subjects that are presenting any symptoms of retinal detachment. Symptoms of retinal detachment include, but are not limited to, presence of floaters in the field of vision, presence of flashes of light, increases in number or frequency of floaters or flashes of light, shadow or curtain over partial field of vision, photoreceptor cell death, loss in peripheral vision, central vision loss, decrease in visual acuity, and blindness. Conversely, GA is a sharply demarcated or complete atrophy of the retinal pigmented epithelium that may or may not involve the fovea. Loss of the RPE results in progressive visual decline.

The compositions described herein are administered topically, intraocularly, intravitreally, subretinally, or systemically. Intraocular administration is preferred. Also preferred are intravitreal and systemic administration. Preferably, the compositions described herein are administered by intraocular injection or systemically. In a preferred embodiment, the composition is administered shortly after diagnosis of retinal detachment, or appearance of a symptom of retinal detachment. In some aspects, the composition is administered within 1 minute, 5 minutes, 10 minutes, 15 minutes, 30 minutes, 45 minutes, 1 hour, 2 hours, 3 hours, 4 hours, 5 hours, 6 hours, 7 hours, 8 hours, 9 hours, 10 hours, 12 hours, 14 hours, 16 hours, 18 hours, 20 hours, 22 hours, or 24 hours, 30 hours, 36 hours, 42, hours, 48 hours, 56 hours or 72 hours after retinal detachment or appearance of a symptom of retinal detachment. Because of the location of the cell death, the inhibitors are preferably delivered subretinally, e.g., using a fine needle for treatment and reduction of cell death associated with retinal detachment.

The composition further comprises a pharmaceutically acceptable carrier and/or ophthalmic excipient. Exemplary pharmaceutically acceptable carrier include a compound selected from the group consisting of a physiological acceptable salt, poloxamer analogs with carbopol, carbopol/hydroxypropyl methyl cellulose (HPMC), carbopol-methyl cellulose, carboxymethylcellulose (CMC), hyaluronic acid, cyclodextrin, and petroleum.

All compounds of the invention are purified and/or isolated. Specifically, as used herein, an "isolated" or "purified" small molecule, nucleic acid molecule, polynucleotide, polypeptide, or protein, is substantially free of other cellular material, or culture medium when produced by recombinant techniques, or chemical precursors or other chemicals when chemically synthesized. Purified compounds are at least 60% by weight (dry weight) the compound of interest. Preferably, the preparation is at least 75%, more preferably at least 90%, and most preferably at least 99%, by weight the compound of interest. For example, a purified compound is one that is at least 90%, 91%, 92%, 93%, 94%, 95%, 98%, 99%, or 100% (w/w) of the desired compound by weight. Purity is measured by any appropriate standard method, for example, by column chromatography, thin layer chromatography, or high-performance liquid chromatography (HPLC) analysis. A purified or isolated polynucleotide (ribonucleic acid (RNA) or deoxyribonucleic acid (DNA)) is free of the genes or sequences that flank it in its naturally occurring state. Purified also defines a degree of sterility that is safe for administration to a human subject, e.g., lacking infectious or toxic agents.

Similarly, by "substantially pure" is meant a nucleotide or polypeptide that has been separated from the components that naturally accompany it. Typically, the nucleotides and polypeptides are substantially pure when they are at least 60%, 70%, 80%, 90%, 95%, or even 99%, by weight, free from the proteins and naturally-occurring organic molecules with they are naturally associated.

An "isolated nucleic acid" is a nucleic acid, the structure of which is not identical to that of any naturally occurring nucleic acid, or to that of any fragment of a naturally occurring genomic nucleic acid spanning more than three separate genes. The term covers, for example: (a) a DNA which is part of a naturally occurring genomic DNA molecule, but is not flanked by both of the nucleic acid sequences that flank that part of the molecule in the genome of the organism in which it naturally occurs; (b) a nucleic acid incorporated into a vector or into the genomic DNA of a prokaryote or eukaryote in a manner, such that the resulting molecule is not identical to any naturally occurring vector or genomic DNA; (c) a separate molecule such as a cDNA, a genomic fragment, a fragment produced by polymerase chain reaction (PCR), or a restriction fragment; and (d) a recombinant nucleotide sequence that is part of a hybridgene, i.e., a gene encoding a fusion protein. Isolated nucleic acid molecules according to the present invention further include molecules produced synthetically, as well as any nucleic acids that have been altered chemically and/or that have modified backbones. Isolated nucleic acid molecules also include messenger ribonucleic acid (mRNA) molecules.

Although the phrase "nucleic acid molecule" primarily refers to the physical nucleic acid and the phrase "nucleic acid sequence" refers to the linear list of nucleotides of the nucleic acid molecule, the two phrases can be used interchangeably.

By the terms "effective amount" and "therapeutically effective amount" of a formulation or formulation component is meant a sufficient amount of the formulation or component, alone or in a combination, to provide the desired effect. For example, by "an effective amount" is meant an amount of a compound, alone or in a combination, required to prevent PVR in a mammal. Ultimately, the attending physician or veterinarian decides the appropriate amount and dosage regimen.

The terms "treating" and "treatment" as used herein refer to the administration of an agent or formulation to a clinically symptomatic individual afflicted with an adverse condition, disorder, or disease, so as to effect a reduction in severity and/or frequency of symptoms, eliminate the symptoms and/or their underlying cause, and/or facilitate improvement or remediation of damage. The terms "preventing" and "prevention" refer to the administration of an agent or composition to a clinically asymptomatic individual who is susceptible or predisposed to a particular adverse condition, disorder, or disease, and thus relates to the prevention of the occurrence of symptoms and/or their underlying cause.

The transitional term "comprising," which is synonymous with "including," "containing," or "characterized by," is inclusive or open-ended and does not exclude additional, unrecited elements or method steps. By contrast, the transitional phrase "consisting of' excludes any element, step, or ingredient not specified in the claim. The transitional phrase "consisting essentially of' limits the scope of a claim to the specified materials or steps "and those that do not materially affect the basic and novel characteristic(s)" of the claimed invention.

Other features and advantages of the invention will be apparent from the following description of the preferred embodiments thereof, and from the claims. Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, suitable methods and materials are described below.

### BRIEF DESCRIPTION OF THE DRAWINGS

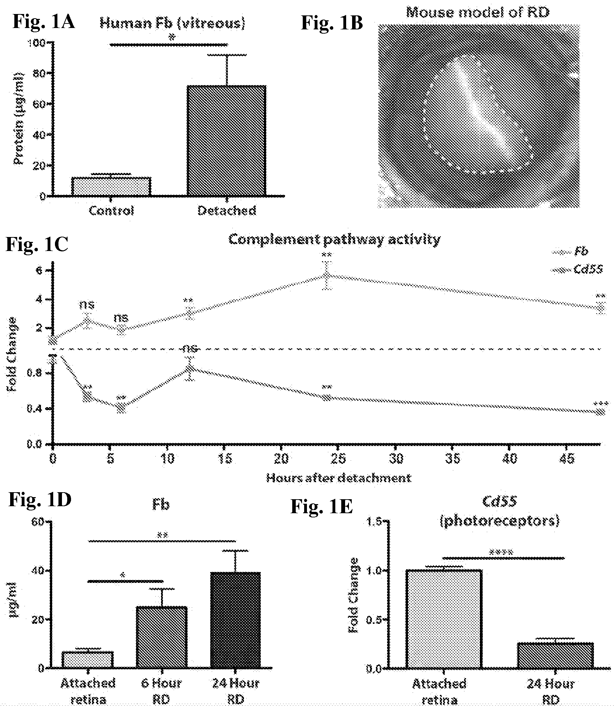

Figs. 1A-E are images and graphs demonstrating the activity of the alternative complement pathway during retinal detachment. Fig. 1A is a bar graph showing results from an ELISA of *Fb* using human vitreous from patients with retinal detachment compared to macular hole as non-detached control samples (control n=4, retinal detachment n=9). Fig. 1B is an image of the mouse model of retinal detachment. The dotted line outlines the region of retina that is detached, approximately 60%. Fig. 1C is a line graph showing a time course for gene expression of *Fb* and *Cd55* in the retina of mice following RD. The line connecting the closed circles tracks *Fb* expression and the line connecting the closed squares tracks *Cd55* expression at intervals over a period of 48 hours. Fig. 1D is a bar graph showing a time course for *Fb* protein following retinal detachment in mice. Fig. 1E is a bar graph showing gene expression of *Cd55* in the ONL 24 hours after RD. (Fb = Factor b, RD = retinal detachment, ONL = outer nuclear layer) (ns = not significant, *≤0.05, **≤0.01, ***≤0.001).

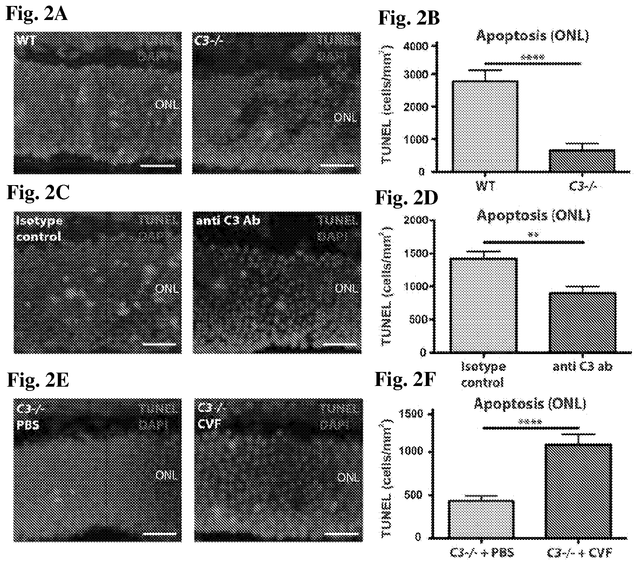

Figs. 2A-F are graphs and images demonstrating apoptosis in complement deficient mice after retinal detachment. Fig. 2A is an image of representative TUNEL labeling in *C3-*/- mice and wild type control (C57BI6) mice 24 hours after retinal detachment (scale bar = 50µm). Fig. 2B is a bar graph depicting quantitation of TUNEL cells in the ONL of *C3-*/*-* mice and wild type control. Fig. 2C is an image of representative TUNEL labeling in the ONL of mice injected with an antibody against C3 compared to IgG control. Fig. 2D is a bar graph depicting quantitation of TUNEL cells in the ONL of mice injected with an antibody against C3 compared to IgG control. Fig. 2E is an image showing representative TUNEL labeling in the ONL of C3-/- mice injected with PBS, control, or CVF (to activate the complement system). Fig. 2F is a bar graph depicting quantitation of TUNEL positive cells in the ONL of *C3-*/*-* mice injected with PBS, control, or CVF (to activate complement in *C3-*/- mice). (WT=wild type, ONL=outer nuclear layer, CVF=cobra venom factor) (**≤0.01, ****≤0.0001, scale bar=50µm).

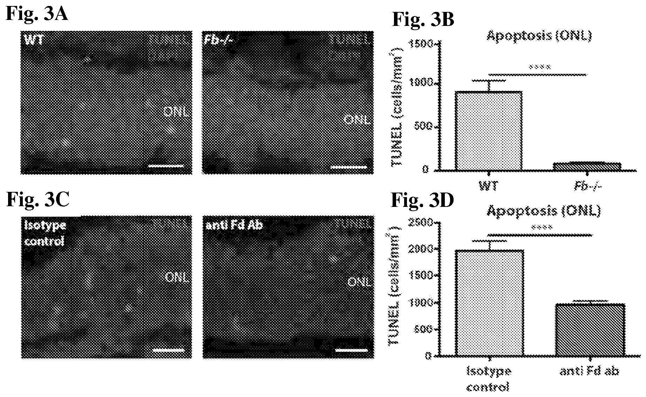

Figs. 3A-D are graphs and images demonstrating apoptosis in alternative pathway deficient mice after retinal detachment. Fig. 3A is an image of representative TUNEL labeling 24 hours after retinal detachment in *Fb-*/*-* mice and wild type control. Fig. 3B is bar graph depicting quantitation of TUNEL positive cells from *Fb-*/*-* mice and wild type control 24 hours after RD. Fig. 3C is an image of representative TUNEL labeling 24 hours after RD in mice injected with a *Fd* neutralizing antibody and IgG isotype control. Fig. 3D is a bar graph depicting quantitation of TUNEL positive cells from mice injected with a *Fd* neutralizing antibody and IgG isotype control 24 hours after RD (WT = wild type, *Fb-*/*-* = Factor b knock out mice, anti Fd ab = antibody against Factor d, RD=retinal detachment, ONL = outer nuclear layer) (****≤0.0001, scale bar = 50µm).

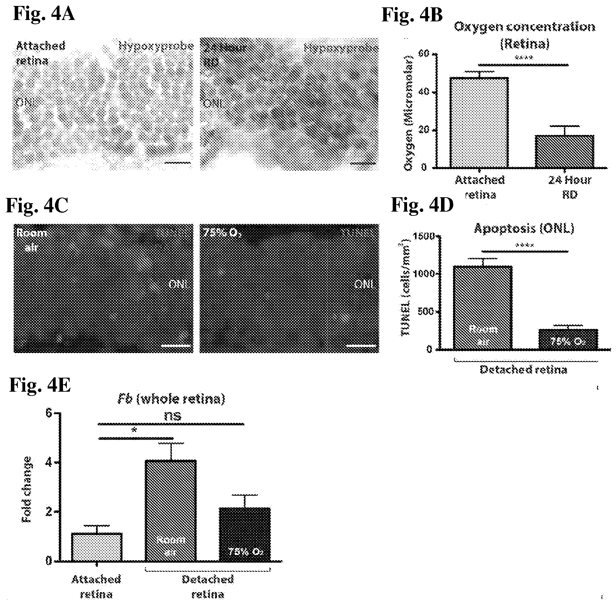

Figs.4A-E are graphs and images demonstrating the role of hypoxia in the retina following retinal detachment. Fig. 4A is an image of representative IHC of the ONL labeled with hypoxyprobe (brown DAB) comparing the detached portion of the retina (right panel) to the attached retina (left panel) in the same retina 24 hours after RD. Fig. 4B is a bar graph of *in vivo* oxygen concentrations taken in the retina 24 hours after RD in an attached retina (right eye) compared to the detached retina (left eye). Fig. 4C is an image of representative TUNEL labeling in the ONL 24 hours after RD in mice kept in room air (left panel) compared to mice kept in 75% oxygen (right panel). Fig. 4D is a bar graph depicting quantitation of TUNEL positive cells in mice kept in room air for 24 hours after RD compared to mice kept in 75% oxygen. Fig. 4E is a bar graph showing RTPCR for gene expression of Fb in the retina 24 hours after RD comparing mice kept in room air compared to mice kept in 75% oxygen. (RD=retinal detachment, ONL=outer nuclear layer) (ns=not significant, *≤0.05, ****≤0.0001, scale bar=50µm).

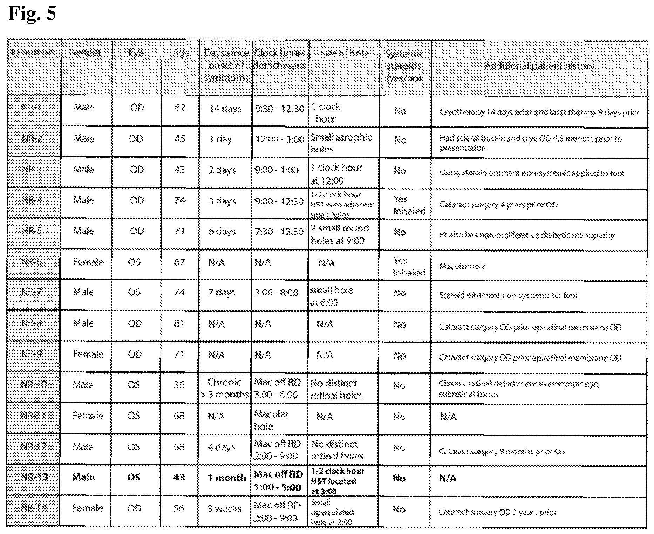

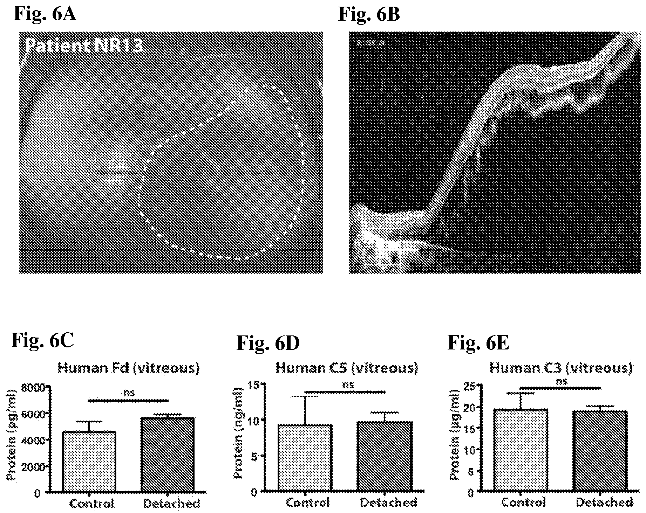

Fig. 5 is a table of human patient data for ELISA samples. Summary of relevant patient history of the samples collected for ELISA of alternative pathway proteins. An OCT of the retinal detachment for patient NR-13, indicated in bold, is shown in Figs 6A.

Figs. 6A-E are graphs and images depicting data from human samples of retinal detachment. Fig. 6A is an image of retinal detachment observed in patient NR13 of Fig. 5. The area outlined by the dotted line is the area of detachment and the solid line the plane of the OCT image shown in Fig. 6B. Fig. 6B is an OCT image taken through the detached area at the plane of the detachment marked by the solid line in Fig. 6A. Fig. 6C is a bar graph of results from an ELISA of complement Factor d. Fig. 6D is a bar graph of results from an ELISA of complement C5. Fig. 6E is a bar graph of results from an ELISA of C3 in the vitreous of patients with a detached retina compared to macular hole, as a non-detached control. (ns=not significant).

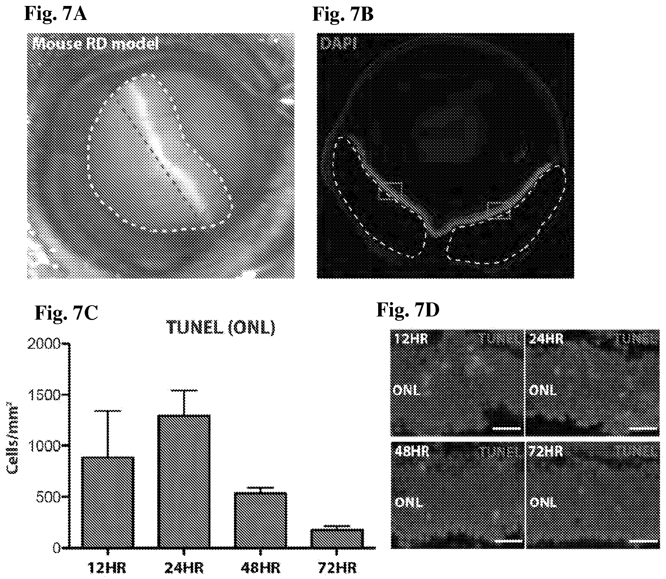

Figs. 7A-D are images and a graph depicting a mouse model of retinal detachment. Fig. 7A is a picture illustrating the typical retinal detachment observed in mice after an injection of provisc® into the sub-retinal space. The dotted white line is outlining the region of the retina that is detached. The dotted black line indicates the cross sectional region in Fig. 7B. Fig. 7B is an image of a cross section of the eye (dotted red line in Fig. 7A) showing the detached retina. The dotted lines outline the detached portion of the retina. The boxes indicate the regions shown in Fig. 7C. Fig. 7C is a bar graph showing a time course of TUNEL labeling in the ONL after retinal detachment. Fig. 7D is an image of TUNEL labeling following a time course after retinal detachment. The peak of apoptosis is at 24 hours. (ONL=outer nuclear layer, scale bar=50µm).

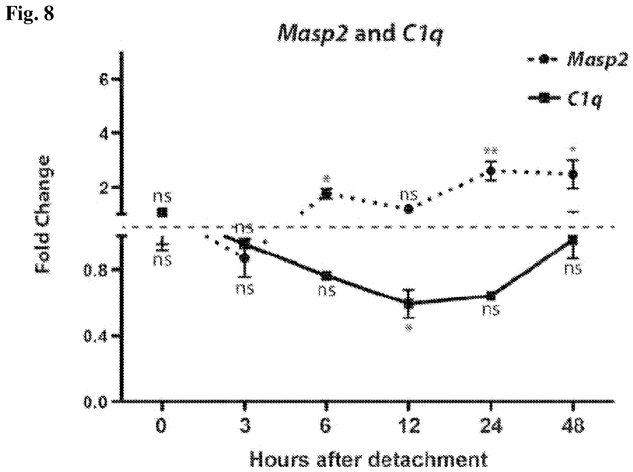

Fig. 8 is a graph demonstrating activation of the lectin and classical complement pathways in the mouse RD model (A) RTPCR showing a time course for *Masp2* (lectin pathway) and *C1q* (classical pathway) gene expression after retinal detachment. (ns=not significant, *≤0.05, **≤0.01).

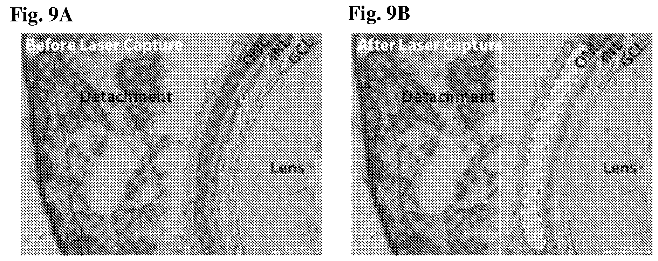

Figs. 9A and B are images showing laser capture micro-dissection of the ONL. Fig. 9A is an image of a cross section of the retina including the detached space stained with toluidine blue dye. Fig. 9B is an image of the same cross section shown in Fig. 9A after cutting out the ONL using laser capture micro-dissection, outlined in a dotted line. (ONL=outer nuclear layer, INL=inner nuclear layer, GCL=ganglion cell layer).

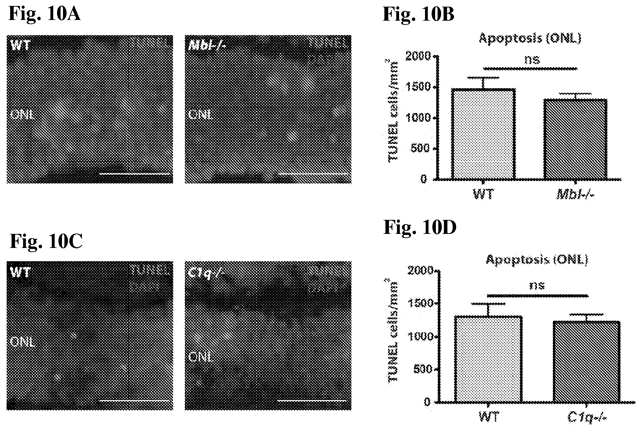

Figs. 10A-D are images and graphs demonstrating the role of the lectin and classical complement pathways in ONL cell death after retinal detachment. Fig. 10A is an image of representative TUNEL labeling 24 hours after RD in *Mbl-*/*-* and wild type, control, mice.

Fig. 10B is a bar graph depicting quantitation of TUNEL positive nuclei in the ONL 24 hours after RD in *MbI-*/*-* and wild type control mice. Fig. 10C is an image of representative TUNEL labeling 24 hours after RD in *C1q-*/*-* and wild type, control, mice. Fig. 10D is a bar graph depicting quantitation of TUNEL positive nuclei in the ONL 24 hours after RD in C1*q-*/- and wild type control mice. (RD=retinal detachment, ONL=outer nuclear layer) (ns=not significant, *≤0.05,**≤0.01, scale bars=50µm).

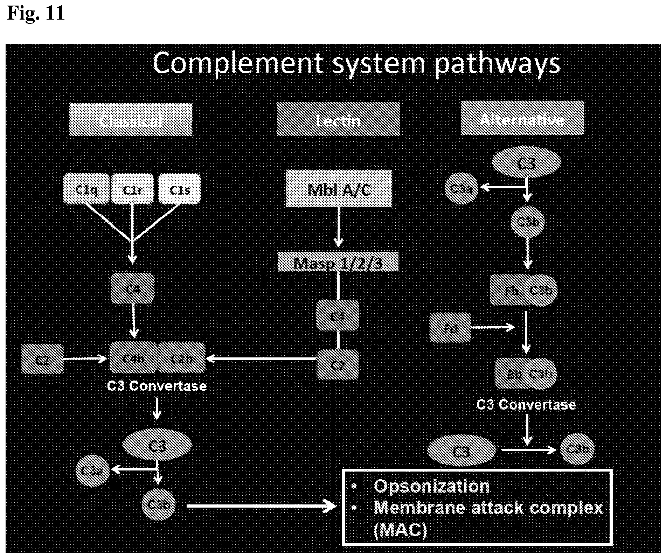

Fig. 11 is a schematic depicting the three complement pathways.

### DETAILED DESCRIPTION

The retina sends visual images to the brain through the optic nerve. Retinal detachment is a disorder of the eye in which the retina peels away from its underlying layer of support tissue. Detachment of the retina causes vision loss and blindness, and if left untreated, the vision loss or blindness can be permanent.

Photoreceptor cell death occurs when the outer segments are physically separated from the underlying retinal pigment epithelium (RPE) and choroidal vasculature (Zacks, D. N., et al, Invest Ophthalmol Vis Sci, 2006, 47:1691-1695). While numerous pathological changes occur in the detached retina, studies in human patient samples and in animal models have shown that photoreceptor cell death is induced as early as 12 hours and peaks at around 2-3 days after RD (Yu, J. et al, Invest Ophalmol Vis Sci, 2012, 53:8146-8153). However, the underlying processes that facilitate this death have remained elusive. Results described herein demonstrate that photoreceptor degeneration correlates with a rise or amplification of the complement system. Without wishing to be bound by theory, the complement system mounts an immune response against the photoreceptors in the damaged retina to specifically target these cells for removal.

The complement system is an intricate innate immune surveillance pathway that is able to discriminate between healthy host tissue, diseased host tissue, apoptotic cells and foreign invaders while modulating the elimination and repair of host tissue accordingly (Yanai, R. et al., Adv Exp Med Biol, 2012, 946:161-183). Consisting of serum and tissue proteins, membrane-bound receptors, and a number of regulatory proteins, the complement system is a hub-like network that is tightly connected to other systems; it comprises three key pathways: the classical, lectin and alternative pathways (Yanai, R. et al., Adv Exp Med Biol, 2012, 946:161-183; and Ricklin, D. et al, Nat Immunol, 2010, 11:785-797). Within the ocular microenvironment the alternative complement pathway exhibits low levels of constitutive activation to ensure the intermittent probing of host self cells, which express inhibitors of complement for protection from activation. On the other hand, damaged or diseased host cells down-regulate membrane-bound inhibitors of complement (CD55), allowing for targeted clearance.

The data described herein demonstrate the surprising results that inhibiting or reducing the alternative complement pathway activity prevent photoreceptor cell death after retinal detachment, thereby resulting in preserving vision. Exemplary agents such as those described in Table 1 below are useful to improve the pathological aspects and symptoms of RD.

**Table 1.**

| DRUG | Mechanism | Route of administration | Phase of clinical trial | COMPANY |
|---|---|---|---|---|
| FCFD4514S | Anti-factor D antibody | Intravitreal | 2^{3,4,6} | Genentech |
| POT-4 (Compstatin) | C3 inhibitor | Intravitreal | 2⁵ | Alcon |
| ARC1905 | Aptamer-based C5 inh | Intravitreal | 1^{1,2} completed | Ophthotec |
| Eculizumab (Soliris) | Anti-C5 antibody | oral | 3 | Alexion. |
| LFG316^{7,8} | Anti-C5 antibody | Intravitreal | | Novartis |
| TA-106 | Complement factor B (CFB) inhibitor | | | Taligen Therapeutics, Alexion Pharmaceuticals |
| | Anti-factor B antibody | | | Genentech |

| | | | | |
|---|---|---|---|---|
| ¹ Opthotech. ClinicalTrials.gov [Internet]. Bethesda (MD): National Library of Medicine (US). 2000 [Cited 2014 Oct 7]. Available from http://clinicaltrials.gov/show/NCT00950638 NLM Identifier: NCT00950638. ²Opthotech. ClinicalTrials.gov [Internet]. Bethesda (MD): National Library of Medicine (US). 2000 [Cited 2014 Oct 7]. Available from http://clinicaltrials.gov/show/NCT00709527 NLM Identifier: NCT00709527. ³ Genentech. ClinicalTrials.gov [Internet]. Bethesda (MD): National Library of Medicine (US). 2000 [Cited 2014 Oct 7]. Available from http://clinicaltrials.gov/show/NCT01229215 NLM Identifier: NCT01229215. ⁴Genentech. ClinicalTrials.gov [Internet]. Bethesda (MD): National Library of Medicine (US). 2000 [Cited 2014 Oct 7]. Available from http://clinicaltrials.gov/show/NCT00973011 NLM Identifier: NCT00973011 ⁵ Potentia Pharmaceuticals, Inc. ClinicalTrials.gov [Internet]. Bethesda (MD): National Library of Medicine (US). 2000 [Cited 2014 Oct 7]. Available from http://clinicaltrials.gov/show/NCT00473928 NLM Identifier: NCT00473928. ⁶ Genentech. ClinicalTrials.gov [Internet]. Bethesda (MD): National Library of Medicine (US). 2000 [Cited 2014 Oct 7]. Available from http://clinicaltrials.gov/show/NCT01602120 NLM Identifier: NCT01602120. ⁷M. Roguska, et al. Generation and Characterization of LFG316, a Fully-Human Anti-C5 Antibody for the Treatment of Age-Related Macular Degeneration [abstract]. In: ARVO 2014 Annual Meeting Abstracts. ARVO Annual Meeting. 2014 May 4-8; Orlando, FL. Abstract No. 3432 - C0281. ⁸A. Carrion, et al. Characterization of the Stoichiometry of Human Complement C5 Binding to LFG316 [abstract]. In: ARVO 2014 Annual Meeting Abstracts. ARVO Annual Meeting. 2014 May 4-8; Orlando, FL. Abstract No. 3432-C0280. | | | | |

Several of the therapies described in the above table are antibodies. To screen for antibodies which bind to a particular epitope on the antigen of interest, a routine cross-blocking assay such as that described in Antibodies, A Laboratory Manual, Cold Spring Harbor Laboratory, Ed Harlow and David Lane (1988), can be performed. Alternatively, epitope mapping, e.g. as described in Champe et al. (1995) J. Biol. Chem. 270:1388-1394, can be performed to determine whether the antibody binds an epitope of interest, as described in WO2009061910A1. Example antibodies from Table 1 above are described briefly below.

### Anti-factor B antibodies

Anti-factor B antibodies are selected using a factor B antigen derived from a mammalian species. Preferably the antigen is human factor B. However, factor Bs from other species such as murine factor B can also be used as the target antigen. The factor B antigens from various mammalian species may be isolated from natural sources. In other embodiments, the antigen is produced recombinantly or made using other synthetic methods known in the art. The antibody selected will normally have a sufficiently strong binding affinity for the factor B antigen. For example, the antibody may bind human factor B with a Kd value of no more than about 5 nM, preferably no more than about 2 nM, and more preferably no more than about 500pM.

Antibody affinities may be determined by a surface plasmon resonance based assay (such as the BiAcore assay as described in Examples); enzyme-linked immunoabsorbent assay (ELISA); and competition assays (e.g. RIA's), for example. Also, the antibody may be subject to other biological activity assays, e.g., in order to evaluate its effectiveness as a therapeutic. Such assays are known in the art and depend on the target antigen and intended use for the antibody. Examples include the HUVEC inhibition assay (as described in the Examples below); tumor cell growth inhibition assays (as described in WO 89/06692, for example); antibody-dependent cellular cytotoxicity (ADCC) and complement-mediated cytotoxicity (CDC) assays (US Patent 5,500,362); and in vitro and in vivo assays described below for identifying factor B antagonists.

To screen for antibodies which bind to a particular epitope on the antigen of interest, a routine cross-blocking assay such as that described in Antibodies, A Laboratory Manual, Cold Spring Harbor Laboratory, Ed Harlow and David Lane (1988), can be performed. Alternatively, epitope mapping, e.g. as described in Champe et al. (1995) J. Biol. Chem. 270:1388-1394, can be performed to determine whether the antibody binds an epitope of interest. In a preferred embodiment, the anti-factor 13 antibodies are selected using a unique phage display approach. The approach involves generation of synthetic antibody phage libraries based on
single framework template, design of sufficient diversities within variable domains, display of polypeptides having the diversified variable domains, selection of candidate antibodies with high affinity to target factor B antigen, and isolation of the selected antibodies.

Examples of anti-factor B antibodies are described in WO2009061910A1, WO2013177035, WO2008140653, and US 20050260198.

### Anti-Factor Bb

An exemplary antibodies specific for factor B for use in inhibiting the activity of C3bBb or PC3bBb complexes, such an antibody inhibits the proteolytic activity of factor B in C3/C5 convertases. Anti-Factor Fb antibodies selectively block the binding of factor Bb to the PC3bB complex without inhibiting classical pathway activation as described in WO2013152020 (incorporated by reference in its entirety herein).

### Anti-factor D

Further example therapies comprise antibodies that target factor D. An example anti-Factor D antibody comprises light chain HVR-1 comprising ITSTDIDDDMN (SEQ ID NO: 1), light chain HVR-2 comprising GGNTLRP (SEQ ID NO: 2), and light chain HVR-3 comprising LQSDSLPYT (SEQ ID NO: 3) and may comprise amino acid substitutions provided that they preserve its antibody binding properties, e.g., as described in US 20140065137. An additional exemplary factor D antibody or a binding fragment thereof may bind to the same epitope on human Factor D as monoclonal antibody 166-32 and is produced by the hybridoma cell line deposited under ATCC Accession Number HB-12476 as described in US 8124090. Another exemplary anti-factor D antibody is US 8372403.

### Anti-C3 Antibody

An example anti C3 antibody selectively blocks the binding of factor B to C3b without inhibiting the classical pathway activation. These types of antibodies, as described in WO2013152024 do not inhibit the interaction of C3b to C5 and therefore have a unique function in inhibiting the alternative pathway. Alternative anti-C3 antibodies can inhibit complement activation by way of inhibition of C3b function, as described in US 20100111946.

### Anti-Properdin Antibody

An exemplary anti-Properdin antibody is that described in WO 2013006449 the epitope comprising amino acids of the sequence: RGRTCRGRKFDGHRCAGQQQDIRHCYSIQHCP (SEQ ID NO: 4).

### Anti-C5 Antibody

The prevention of C5a generation with antibodies during the arrival of sepsis in rodents has been shown to greatly improve survival, while related findings were made when the C5a receptor (C5aR) was blocked, using either antibodies or a small molecular inhibitor (Laudes, U., et al., Anti-c5a ameliorates coagulation/fibrinolytic protein changes in a rat model of sepsis. American Journal Of Pathology, 2002. 160(5): p. 1867; Riedemann, N. C., R. F. Guo, and P. A. Ward, A key role of C5a/C5aR activation for the development of sepsis. Journal of Leukocyte Biology, 2003. 74(6): p. 966). An additional exemplary anti-C5 antibody is described in WO1995029697. An additional example antibody is the monoclonal antibody designated MAb137-26, which binds to a shared epitope of human C5 and C5a, as described in US8372404.

Non antibody inhibitors are also listed in Table 1 above, brief descriptions of these inhibitors are found below.

### POT-4

POT-4 is a derivative of the cyclic peptide Compostatin. It is capable of binding to human complement factor C3 (Potentia pharmaceuticals, http://www.potentiapharma.com/products/pot4.htm). POT 4 suppresses complement activation by preventing the formation of key elements within the proteolytic cascade, thus impeding local inflammation, upregulation of angiogenic factors and subsequent tissue damage (O. S. Punjabi and P.K. Kaiser, Review of Ophthalmology. Oct 4, 2012. http://www.reviewofophthalmology.com/content/d/retinal_insider/c/36952).

### ARC 1905

ARC 1905 is a PEGylated, stabilized aptamer targeting complement factor C5, blocking the cleavage of C5 into C5a and C5b fragments (ARC1905 inhibits C5 - Dry/Wet AMD Intravitreal, http://www.amdbook.org/content/arc1905-inhibits-c5-drywet-amd-intravitreal). Like POT-4, it is similarly selective for a centrally positioned component within the cascade, although exerting its effect further downstream (O. S. Punjabi and P.K. Kaiser, Review of Opthomology. Oct 4, 2012. http://www.reviewofophthalmology.com/content/d/retinal_insider/c/36952).

### TA-106

TA-106 inhibits Factor B, a serine proteinase that is unique to the alternative pathway and exists upstream of complement proteins targeted by many other drugs, including complement 3 (C3) and C5, (BioCentury, The Bernstein Report on BioBusiness. August 13, 2007). It is primarily being investigated as an inhaled formulation in the treatment of severe, chronic asthma refractory to current therapies and is recently being studied for macular degeneration (O. S. Punjabi and P.K. Kaiser, Review of Ophthalmology. Oct 4, 2012. http://www.reviewofophthalmology.com/content/d/retinal_insider/c/36952).

Many of the therapies described above were developed for the treatment of Age-related Macular Dystrophy (AMD). However, data described herein demonstrates the applicability of inhibitors of the alternative complement pathway in the treatment of retinal detachment.

The methods and compositions described herein provide a non-surgical therapy for retinal detachment.

### Retinal Detachment

There are three types of retinal detachment: rhegmatogenous, exudative, and tractional. Rhegmatogenous retinal detachment occurs due to a break in the retina that allows fluid to pass from the vitreous space into the subretinal space between the sensory retina and the retinal pigment epithelium. Chronic retinal atrophy or a result of vitreous traction can cause retinal breaks. Retinal breaks include retinal holes, retinal tears, and retinal dialyses. Exudative retinal detachment (also known as serous or secondary retinal detachment) occurs due to inflammation, injury or vascular abnormalities that result in fluid accumulating underneath the retina without the presence of a hole, tear, or break. In some rare cases, exudative retinal detachment can be caused by the growth of a tumor on the layers of tissue beneath the retina (*i.e.,* choroidal melanoma). Tractional retinal detachment occurs when fibrous or fibrovascular tissue pulls the sensory retina from the retinal pigment epithelium, often caused by injury, inflammation, or neovascularization.

The compositions and methods described herein are particularly useful for treating a subject that has suffered from a trauma-induced retinal detachment. Trauma-induced retinal detachment, as used herein, refers to a retinal detachment resulting from a physical trauma or injury, for example, blunt or penetrating blows to the eye or areas surrounding the eye, concussion to the head, or previous eye surgery. Trauma-induced retinal detachment can occur in high impact sports (*i.e.,* boxing, karate, kickboxing, American football, high intensity weightlifting), in high speed sports (*i.e.,* automobile racing, sledding), or in activities that increase pressure on or within the eye itself, or include rapid acceleration and deceleration. Active military personnel are also at increased risk for retinal detachment, for example, as a result of injuries sustained from improvised explosive devices (IEDs) blasts in the field.

Symptoms of retinal detachment include presence of flashes of light (photopsia), floaters, or a shadow or curtain in the field of vision. The flashes of light may be sudden, and very brief in the extreme peripheral part of the vision. Floaters may look like small debris, spots, hairs, or string that float in the field of vision. The shadow or curtain may appear in a portion of the field of vision, and develops or spreads as the detachment progresses. Other symptoms include sudden or dramatic increase in the number of floaters or flashes of light. Other symptoms include a slight feeling of heaviness in the eye, central visual loss, decrease in visual acuity, any vision loss, or blindness.

Retinal detachment can occur at any age, but it is more common in midlife and later. Conditions that can increase the chance of a retinal detachment include, for example, nearsightedness (or severe myopia), previous cataract surgery, severe trauma, previous retinal detachment in either eye, family history of retinal detachment, proliferative diabetic retinopathy, retinopathy of prematurity, eclampsia, homocysteinuria, malignant hypertension, inflammatory conditions (*i.e.,* uveitis or scleritis), glaucoma, retinoblastoma, metastatic cancer that spreads to the eye, choroidal melanoma, haemangioma, Stickler syndrome, Von Hippel-Lindau disease, AIDS, and smoking.

Diagnosis of retinal detachment is performed using fundus photography, ophthalmography, or medical ultrasonography.

Generally, current treatment methods for treating retinal detachment aim to find and seal all retinal breaks, and relieve present and future vitreoretinal traction. These treatment procedures include, but are not limited to, cryopexy (freezing) and laser photocoagulation, scleral buckle surgery, pneumatic retinopexy, and vitrectomy.

The compositions and methods described herein provide the advantage over the current treatment methods by being non-surgical, and having less associated complications. The compositions and methods disclosed herein prevent photoreceptor cell death, thereby allowing the retinal to reattach post-injury. Furthermore, administration of the compositions disclosed herein is useful for preventing progression of retinal detachment when delivered shortly after diagnosis of retinal detachment or appearance of a symptom of retinal detachment. For example, the composition is administered within 6 hours, 12 hours, 18 hours, 24 hours, 30 hours, 36 hours, 42, hours, 48 hours, 56 hours or 72 hours after retinal detachment or appearance of a symptom of retinal detachment. Methods described herein can also be performed in combination with any of the current treatment methods or surgical procedures used to treat retinal detachment or reattach the detached retina.

### Complement Pathway

The complement system is comprised of various soluble and surface-bound complement components, receptor and regulators that are initiated by interaction of several pattern-recognition receptors with foreign surface structures. Depending on the activation trigger, the complement cascade follows one of three pathways: the classical, lectin or alternative pathway. The three complement pathways follow a different sequence of "early" cleavage reactions that all lead to the assembly of a protease named C3 convertase. The C3 convertase then cleaves protein C3 into C3a and C3b, which in turn causes a cascade of signaling and cleavage of other complement components, eventually initiating the formation of the membrane attack complex (MAC). MAC comprises C5b, C6, C7, C8, and polymeric C9, forming a transmembrane channel that causes osmotic lysis of the target cell. A schematic of the pathways and their components is shown in Fig. 11.

The alternative pathway is initiated by the hydrolysis of circulating C3 to expose an internal thioester group. This phenomenon is referred to as "C3 tickover". The hydrolysed C3 then binds alternative-pathway-specific proteins factor B, factor D, and properdin, to form activated C3-convertase. At this point, the alternative complement pathway converges with the classical and lectin pathways in the cleavage of C3 into active fragments of C3a and C3b by the C3 convertases. In an amplification loop, factor B can also bind C3b and is processed by factor D to generate more C3 convertase, thereby initiating an acceleration of C3b production. C3b initiates the formation of the two C5 convertases, which cleaves C5 to C5a and C5b. C5b initiates the assembly of the membrane attack complex (MAC), a pore that is formed by components C5b, C6, C7, C8, and multiple units of C9 (C5b-9), and ultimately leads to cell lysis and death.

The lectin pathway is activated by binding of mannose-binding lectin (MBL) to mannose residues on the pathogen surface, which activates the MBL-associated serine proteases, MASP-1, and MASP-2 (very similar to C1r and C1s, respectively), which can then split C4 into C4a and C4b and C2 into C2a and C2b. C4b and C2b then bind together to form C3-convertase. RT-PCR analysis of components of the lectin pathway in models of retinal detachment show that the lectin complement pathway is also highly regulated in retinal detachment.

The methods and compositions disclosed herein inhibit or reduce the activity of a complement pathway. Preferably, the complement pathway is the alternative complement pathway. Inhibiting or reducing the activity of a complement pathway, as used herein, refers to the modulation the transcription stability, translation, modification, localization, cleavage, or function of a polynucleotide or polypeptide encoding any one of the selected from properdin (Factor p), factor B, factor Ba, factor Bb, factor D, C2, C2a, C3a, C5, C5a, C6, C7, C8, C9, and C5b-9. Preferably, the modulation results in an inhibition or decrease in the activity (*i.e.,* function) or expression of the complement component.

In some embodiments, the agent that inhibits or reduces complement pathway activity comprises an antibody or an antibody fragment. Preferably, the antibody specifically binds to the properdin (Factor p), factor B, factor Ba, factor Bb, factor D, C2, C2a, C3a, C3b, C5, C5a, C5b, C6, C7, C8, C9, and C5b-9. In a preferred embodiment, the antibody inhibits the activity (function) of the complement component, for example, preventing or reducing the binding to the cognate receptor, the binding of the receptor to the ligand, cleavage, or activation. Examples of therapeutic antibodies include eculizumab, pexelizumab, ofatumumab, TNX-234, TNX-558, TA106, neutrazumab, and anti-properdin. An exemplary antibody specifically binds to factor B (Fb).

The term "antibody" as used herein includes whole antibodies and any antigen binding fragment (i. e., "antigen-binding portion") or single chains thereof. A naturally occurring "antibody" is a glycoprotein comprising at least two heavy (H) chains and two light (L) chains inter-connected by disulfide bonds. Each heavy chain is comprised of a heavy chain variable region (abbreviated herein as VH) and a heavy chain constant region. The heavy chain constant region is comprised of three domains. CH1, CH2 and CH3. Each light chain is comprised of a light chain variable region (abbreviated herein as VL) and a light chain constant region. The light chain constant region is comprised of one domain, CL. The VH and VL regions can be further subdivided into regions of hypervariability, termed complementarity determining regions (CDR), interspersed with regions that are more conserved, termed framework regions (FR). Each VH and VL is composed of three CDRs and four FRs arranged from amino-terminus to carboxy-terminus in the following order: FR1, CDR1, FR2, CDR2, FR3, CDR3, FR4. The variable regions of the heavy and light chains contain a binding domain that interacts with an antigen. The constant regions of the antibodies may mediate the binding of the immunoglobulin to host tissues or factors, including various cells of the immune system (e.g., effector cells) and the first component (C1q) of the classical complement system.

"Humanized" forms of non-human (e.g., murine) antibodies are chimeric antibodies which contain minimal sequence derived from non-human immunoglobulin. For the most part, humanized antibodies are human immunoglobulins (recipient antibody) in which hypervariable region residues of the recipient are replaced by hypervariable region residues from a non-human species (donor antibody) such as mouse, rat, rabbit or nonhuman primate having the desired specificity, affinity, and capacity. In some instances, framework region (FR) residues of the human immunoglobulin are replaced by corresponding non-human residues. Furthermore, humanized antibodies may comprise residues which are not found in the recipient antibody or in the donor antibody. These modifications are made to further refine antibody performance. In general, the humanized antibody will comprise substantially all of at least one, and typically two, variable domains, in which all or substantially all of the hypervariable regions correspond to those of a non-human immunoglobulin and all or substantially all of the FRs are those of a human immunoglobulin sequence. The humanized antibody optionally also will comprise at least a portion of an immunoglobulin constant region (Fc), typically that of a human immunoglobulin. For further details, see Jones et al., Nature 321:522-525 (1986); Reichmann et al., Nature 332:323-329 (1988); and Presta, Curr. Op. Struct. Biol. 2:593-596 (1992).

Human monoclonal antibodies can be prepared by using trioma technique; the human B-cell hybridoma technique (Kozbor, et al., 1983 Immunol Today 4: 72); and the EBV hybridoma technique to produce human monoclonal antibodies (Cole, et al., 1985 In: Monoclonal Antibodies and Cancer Therapy, Alan R. Liss, Inc., pp. 77-96). Human monoclonal antibodies may be utilized and may be produced by using human hybridomas (Cote, et al., 1983. Proc Natl Acad Sci USA 80: 2026-2030) or by transforming human B-cells with Epstein Barr Virus *in vitro* (Cole, et al., 1985 In: Monoclonal Antibodies and Cancer Therapy, Alan R. Liss, Inc., pp. 77-96). In addition, human antibodies can also be produced using additional techniques, including phage display libraries. (Hoogenboom and Winter, J. Mol. Biol., 227:381 (1991); Marks et al., J. Mol. Biol., 222:581 (1991)). Similarly, human antibodies can be made by introducing human immunoglobulin loci into transgenic animals, e.g., mice in which the endogenous immunoglobulin genes have been partially or completely inactivated. Upon challenge, human antibody production is observed, which closely resembles that seen in humans in all respects, including gene rearrangement, assembly, and antibody repertoire. This approach is described, for example, in U.S. Patent Nos. 5,545,807; 5,545,806; 5,569,825; 5,625,126; 5,633,425; 5,661,016, and in Marks et al., Bio/Technology 10, 779-783 (1992); Lonberg et al., Nature 368 856-859 (1994); Morrison, Nature 368, 812-13 (1994); Fishwild et al., Nature Biotechnology 14, 845-51 (1996); Neuberger, Nature Biotechnology 14, 826 (1996); and Lonberg and Huszar, Intern. Rev. Immunol. 13 65-93 (1995).

Human antibodies may additionally be produced using transgenic nonhuman animals which are modified so as to produce fully human antibodies rather than the animal's endogenous antibodies in response to challenge by an antigen (PCT publication WO94/02602). The endogenous genes encoding the heavy and light immunoglobulin chains in the nonhuman host have been incapacitated, and active loci encoding human heavy and light chain immunoglobulins are inserted into the host's genome. The human genes are incorporated, for example, using yeast artificial chromosomes containing the requisite human DNA segments. An animal which provides all the desired modifications is then obtained as progeny by crossbreeding intermediate transgenic animals containing fewer than the full complement of the modifications. The preferred embodiment of such a nonhuman animal is a mouse, and is termed the Xenomouse™ as disclosed in PCT publications WO 96/33735 and WO 96/34096. This animal produces B cells which secrete fully human immunoglobulins. The antibodies can be obtained directly from the animal after immunization with an immunogen of interest, as, for example, a preparation of a polyclonal antibody, or alternatively from immortalized B cells derived from the animal, such as hybridomas producing monoclonal antibodies. Additionally, the genes encoding the immunoglobulins with human variable regions can be recovered and expressed to obtain the antibodies directly, or can be further modified to obtain analogs of antibodies such as, for example, single chain Fv (scFv) molecules.

Services are currently offered commercially by companies (*i.e.,* Immunomedics Inc., 300 The American Road, Morris Plains, NJ 07950, USA; Antitope Ltd., Babraham Research Campus, Babraham, Cambridge CB22 3AT, United Kingdom; and GenScript USA Inc., 860 Centennial Ave., Piscataway, NJ 08854, USA) for the production of humanized antibodies.

The term "antigen binding portion" of an antibody, as used herein, refers to one or more fragments of an intact antibody that retain the ability to specifically bind to a given antigen (e.g., C3b). Antigen binding functions of an antibody can be performed by fragments of an intact antibody. Examples of binding fragments encompassed within the term "antigen binding portion" of an antibody include a Fab fragment, a monovalent fragment consisting of the VL, VH, CL and CH1 domains; a F(ab)₂ fragment, a bivalent fragment comprising two Fab fragments linked by a disulfide bridge at the hinge region; an Fd fragment consisting of the VH and CH1 domains; an Fv fragment consisting of the VL and VH domains of a single arm of an antibody; a single domain antibody (dAb) fragment (Ward et al.. 1989 Nature 341:544-546), which consists of a VH domain or a VL domain; and an isolated complementarity determining region (CDR).

Furthermore, although the two domains of the Fv fragment, VL and VH, are coded for by separate genes, they can be joined, using recombinant methods, by an artificial peptide linker that enables them to be made as a single protein chain in which the VL and VH regions pair to form monovalent molecules (known as single chain Fv (scFv); see, e.g., Bird et al., 1988 Science 242:423-426; and Huston et al., 1988 Proc. Natl. Acad. Sci. 65:5879-5883). Such single chain antibodies include one or more "antigen binding portions" of an antibody. These antibody fragments are obtained using conventional techniques known to those of skill in the art, and the fragments are screened for utility in the same manner as are intact antibodies. Antigen binding portions can also be incorporated into single domain antibodies, maxibodies, minibodies, interbodies, diabodies, triabodies, totrabodies. v-NAR and bis-scFv (see, e.g., Hollinger and Hudson. 2005. Nature Biotechnology, 23, 9, 1126-1136).

Antigen binding portions can be incorporated into single chain molecules comprising a pair of tandem Fv segments (VH-CH1-VH-CH1) which, together with complementary light chain polypeptides, form a pair of antigen binding regions (Zapata et al., 1995 Protein Eng. 8(10): 1057- 1062; and U.S. Pat. No. 5,641,870).

The term "binding specificity" or "specifically binds" as used herein refers to the ability of an individual antibody combining site to react with only one antigenic determinant, and therefore does not bind other complement components. The combining site of the antibody is located in the Fab portion of the molecule and is constructed from the hypervariable regions of the heavy and light chains. Binding affinity of an antibody is the strength of the reaction between a single antigenic determinant and a single combining site on the antibody. It is the sum of the attractive and repulsive forces operating between the antigenic determinant and the combining site of the antibody.

Other agents that inhibit or reduce complement pathway activity are serine protease inhibitors. The complement cascade relies upon the consecutive cleavage and activation of several proteases. Proteases in the complement cascade include, for example, C1r, C1s, C2a, MASP1, MASP2, factor D, and factor B. The protease inhibitor binds to the protease and preferably prevents its cleavage function. Examples of serine protease inhibitors are C1-Inh and rhucin.

Soluble complement regulators are also useful as agents that inhibit or reduce complement pathway activity. For example, the agent is a soluble form of a complement receptor that competes with endogenous complement receptors, thereby reducing the complement pathway activity. Alternatively, the agent is a soluble form of an endogenous complement inhibitor that reduces complement pathway activity. For example, the agent is a soluble form of DAF/CD55 of CD59. Examples of soluble complement regulators include sCR1/TP10, CAB-2/MLN-2222, mirococept, and soluble CD55 mimetic.

Another agent that inhibits or reduces complement pathway activity is a complement component inhibitor, such as a small molecule that interrupts protein functions by steric hindrance or induction of conformational changes. The agent may be a peptide, a nucleotide, or a synthetic molecule. For example, the agent may be an aptamer, which is a single-stranded nucleotide that has molecular recognition properties similar to those of antibodies but can be selected in an automated high-throughput process known as SELEX. Aptamers that recognize complement components can provide complete blockage of downstream complement activation. For example, anti-C5 aptamer (ARC 1905) features a subnanomolar binding affinity for C5 and inhibits the cleavage of C5a and C5b. Other examples of complement component inhibitors include compstatin/POT-4.

Anaphylatoxin receptor antagonists can also be used to inhibit or reduce complement cascade signaling. Anaphylatoxins C3a and C5a are potent inflammatory mediators that binds to high affinity receptors. These antagonists are designed to bind to the receptor with high affinity without inducing any signaling activity, thereby inhibiting and reducing complement pathway activity. Examples of anaphylatoxin receptor antagonists include PMX-53, PMX-205, JPE-1375, and JSM-7717.

Nucleic acid expression vectors that encodes an anti-complement agent are also useful for inhibiting or reducing complement pathway activity. The anti-complement agent may be a polynucleotide or a polypeptide that inhibits or reduces a complement component activity or expression. The polynucleotide may be an interfering RNA or an aptamer. The polypeptide may be a complement inhibitor or receptor antagonist. For example, the agent is an AAV-expression vector comprising CD55.

Any of the agents described herein may be derivatized or modified using methods known in the art for modulating the pharmacological properties, such as stability, half-life, permeability, and affinity.

### Pharmaceutical Compositions

For administration to a subject such as a human or other mammal (e.g., companion, zoological or livestock animal), an agent that inhibits or reduces alternative complement pathway activity is desirably formulated into a pharmaceutical composition containing the active agent in admixture with one or more pharmaceutically acceptable diluents, excipients or carriers. Examples of such suitable excipients for can be found in U.S. Publication 2009/0298785, the Handbook of Pharmaceutical Excipients, 2nd Edition (1994), Wade and Weller, eds. Acceptable carriers or diluents for therapeutic use are well-known in the pharmaceutical art, and are described, for example, in Remington: The Science and Practice of Pharmacy, 20th Edition (2000) Alfonso R. Gennaro, ed., Lippincott Williams & Wilkins: Philadelphia, Pa. Examples of suitable carriers include lactose, starch, glucose, methyl cellulose, magnesium stearate, mannitol, sorbitol and the like. Examples of suitable diluents include ethanol, glycerol and water. The choice of pharmaceutical carrier, excipient or diluent can be selected with regard to the intended route of administration and standard pharmaceutical practice. The pharmaceutical composition can contain as, or in addition to, the carrier, excipient or diluent any buffering agent(s), suitable binder(s), lubricant(s), suspending agent(s), coating agent(s), solubilizing agent(s), isotonifier(s), non-ionic detergent(s), and other miscellaneous additives. Such additives must be nontoxic to the recipients at the dosages and concentrations employed.

Buffering agents help to maintain the pH in the range which approximates physiological conditions. They are preferably present at concentration ranging from about 2 mM to about 50 mM. Suitable buffering agents for use with the present invention include both organic and inorganic acids and salts thereof such as citrate buffers (e.g., monosodium citrate-disodium citrate mixture, citric acid-trisodium citrate mixture, citric acid-monosodium citrate mixture, etc.), succinate buffers (e.g., succinic acid-monosodium succinate mixture, succinic acid-sodium hydroxide mixture, succinic acid-disodium succinate mixture, etc.), tartrate buffers (e.g., tartaric acid-sodium tartrate mixture, tartaric acid-potassium tartrate mixture, tartaric acid-sodium hydroxide mixture, etc.), fumarate buffers (e.g., fumaric acid-monosodium fumarate mixture, etc.), fumarate buffers (e.g., fumaric acid-monosodium fumarate mixture, fumaric acid-disodium fumarate mixture, monosodium fumarate-disodium fumarate mixture, etc.), gluconate buffers (e.g., gluconic acid-sodium glyconate mixture, gluconic acid-sodium hydroxide mixture, gluconic acid-potassium glyuconate mixture, etc.), oxalate buffer (e.g., oxalic acid-sodium oxalate mixture, oxalic acid-sodium hydroxide mixture, oxalic acid-potassium oxalate mixture, etc.), lactate buffers (e.g., lactic acid-sodium lactate mixture, lactic acid-sodium hydroxide mixture, lactic acid-potassium lactate mixture, etc.) and acetate buffers (e.g., acetic acid-sodium acetate mixture, acetic acid-sodium hydroxide mixture, etc.). Additionally, there may be mentioned phosphate buffers, histidine buffers and trimethylamine salts such as Tris.

Examples of suitable binders include starch, gelatin, natural sugars such as glucose, anhydrous lactose, free-flow lactose, beta-lactose, corn sweeteners, natural and synthetic gums, such as acacia, tragacanth or sodium alginate, carboxymethyl cellulose and polyethylene glycol.

Examples of suitable lubricants include sodium oleate, sodium stearate, magnesium stearate, sodium benzoate, sodium acetate, sodium chloride and the like.

Preservatives, stabilizers, dyes and even flavoring agents can be provided in the pharmaceutical composition. Examples of preservatives include sodium benzoate, sorbic acid and esters of p-hydroxybenzoic acid. Antioxidants and suspending agents can be also used. Preservatives may be added to retard microbial growth, and may be added in amounts ranging from 0.2%-1% (w/v). Suitable preservatives for use with the present invention include phenol, benzyl alcohol, meta-cresol, methyl paraben, propyl paraben, octadecyldimethylbenzyl ammonium chloride, benzalconium halides (e.g., chloride, bromide, iodide), hexamethonium chloride, alkyl parabens such as methyl or propyl paraben, catechol, resorcinol, cyclohexanol, and 3-pentanol.

Isotonicifiers sometimes known as "stabilizers" may be added to ensure isotonicity of liquid compositions of the present invention and include polhydric sugar alcohols, preferably trihydric or higher sugar alcohols, such as glycerin, erythritol, arabitol, xylitol, sorbitol and mannitol.

Stabilizers refer to a broad category of excipients which can range in function from a bulking agent to an additive which solubilizes the therapeutic agent or helps to prevent denaturation or adherence to the container wall. Typical stabilizers can be polyhydric sugar alcohols (enumerated above); amino acids such as arginine, lysine, glycine, glutamine, asparagine, histidine, alanine, ornithine, L-leucine, 2-phenylalanine, glutamic acid, threonine, etc., organic sugars or sugar alcohols, such as lactose, trehalose, stachyose, mannitol, sorbitol, xylitol, ribitol, myoinisitol, galactitol, glycerol and the like, including cyclitols such as inositol; polyethylene glycol; amino acid polymers; sulfur containing reducing agents, such as urea, glutathione, thioctic acid, sodium thioglycolate, thioglycerol, α-monothioglycerol and sodium thio sulfate; low molecular weight polypeptides (i.e. <10 residues); proteins such as human serum albumin, bovine serum albumin, gelatin or immunoglobulins; hydrophylic polymers, such as polyvinylpyrrolidone monosaccharides, such as xylose, mannose, fructose, glucose; disaccharides such as lactose, maltose, sucrose and trisaccacharides such as raffinose; polysaccharides such as dextran. Stabilizers may be present in the range from 0.1 to 10,000 weights per part of weight active protein.

Non-ionic surfactants or detergents (also known as "wetting agents") may be added to help solubilize the therapeutic agent as well as to protect the therapeutic protein against agitation-induced aggregation, which also permits the formulation to be exposed to shear surface stressed without causing denaturation of the protein. Suitable non-ionic surfactants include polysorbates (20, 80, etc.), polyoxamers (184, 188 etc.), Pluronic® polyols, polyoxyethylene sorbitan monoethers (Tween®-20, Tween®-80, etc.). Non-ionic surfactants may be present in a range of about 0.05 mg/ml to about 1.0 mg/ml, preferably about 0,07 mg/ml to about 0.2 mg/ml.

Additional miscellaneous excipients include bulking agents, (e.g. starch), chelating agents (e.g. EDTA), antioxidants (e.g., ascorbic acid, methionine, vitamin E), and cosolvents. The formulation herein may also contain more than one active compound as necessary for the particular indication being treated, preferably those with complementary activities that do not adversely affect each other. For example, it may be desirable to further provide an immunosuppressive agent. Such molecules are suitably present in combination in amounts that are effective for the purpose intended. The active ingredients may also be entrapped in microcapsule prepared, for example, by coascervation techniques or by interfacial polymerization, for example, hydroxymethylcellulose or gelatin-microcapsule and poly-(methylmethacylate) microcapsule, respectively, in colloidal drug delivery systems (for example, liposomes, albumin micropheres, microemulsions, nano-particles and nanocapsules) or in macroemulsions, Such techniques are disclosed in Remington's Pharmaceutical Sciences, 16th edition, A. Osal, Ed. (1980).

The formulations to be used for in vivo administration must be sterile. This is readily accomplished, for example, by filtration through sterile filtration membranes. Sustained-release preparations may be prepared. Suitable examples of sustained-release preparations include semi-permeable matrices of solid hydrophobic polymers containing the antibody variant, which matrices are in the form of shaped articles, e.g., films, or microcapsules. Examples of sustained-release matrices include polyesters, hydrogels (for example, poly(2-hydroxyethyl-methacrylate), or poly(vinylalcohol)), polylactides (U.S. Pat. No. 3,773,919), copolymers of L-glutamic acid and ethyl-L-glutamate, non-degradable ethylene-vinyl acetate, degradable lactic acid-glycolic acid copolymers such as the LUPRON DEPOT™ (injectable microspheres composed of lactic acid-glycolic acid copolymer and leuprolide acetate), and poly-D-(-)-3-hydroxybutyric acid. While polymers such as ethylene-vinyl acetate and lactic acid-glycolic acid enable release of molecules for over 100 days, certain hydrogels release proteins for shorter time periods.

The active ingredients may also be entrapped in microcapsule prepared, for example, by coacervation techniques or by interfacial polymerization, for example, hydroxymethylcellulose or gelatin-microcapsule and poly-(methylmethacylate) microcapsule, respectively, in colloidal drug delivery systems (for example, liposomes, albumin microspheres, microemulsions, nano-particles and nanocapsules) or in macroemulsions. Such techniques are disclosed in Remington's Pharmaceutical Sciences 16th edition, Osol, A. Ed. (1980).

Sustained-release preparations may be prepared. Suitable examples of sustained-release preparations include semipermeable matrices of solid hydrophobic polymers containing the anti-complement inhibitors, which matrices are in the form of shaped articles, *e.g.,* films, or microcapsule. Examples of sustained-release matrices include polyesters, hydrogels (for example, poly(2-hydroxyethyl-methacrylate), or poly(vinylalcohol)), polylactides (U.S. Pat. No, 3,773,919), copolymers of L-glutamic acid and γ ethyl-L-glutamate, non-degradable ethylene-vinyl acetate, degradable lactic acid-glycolic acid copolymers such as the Lupron Depot™ (injectable microspheres composed of lactic acid-glycolic acid copolymer and leuprolide acetate), and poly-D-(-)-3-hydroxybutync acid. While polymers such as ethylene-vinyl acetate and lactic acid--glycolic acid enable release of molecules for over 100 days, certain hydrogels release proteins for shorter time periods. When encapsulated antibodies remain in the body for a long time, they may denature or aggregate as a result of exposure to moisture at 37°C, resulting in a loss of biological activity and possible changes in immunogenicity. Rational strategies can be devised for stabilization depending on the mechanism involved. For example, if the aggregation mechanism is discovered to be intermolecular S-S bond formation through thio-disulfide interchange, stabilization may be achieved by modifying sulfhydryl residues, lyophilizing from acidic solutions, controlling moisture content, using appropriate additives, and developing specific polymer matrix compositions.

A suitable formulation of the compositions disclosed herein is a hydrogel. A hydrogel is a colloidal gel formed as a dispersion in water or other aqueous medium. Thus a hydrogel is formed upon formation of a colloid in which a dispersed phase (the polymer) has combined with a continuous phase (i.e. water) to produce a viscous jellylike product; for example, coagulated silicic acid. A hydrogel is a three-dimensional network of hydrophilic polymer chains that are crosslinked through either chemical or physical bonding. Because of the hydrophilic nature of the polymer chains, hydrogels absorb water and swell (unless they have already absorbed their maximum amount of water). The swelling process is the same as the dissolution of non-crosslinked hydrophilic polymers. By definition, water constitutes at least 10% of the total weight (or volume) of a hydrogel,

Examples of hydrogels include synthetic polymers such as polyhydroxy ethyl methacrylate, and chemically or physically crosslinked polyvinyl alcohol, polyacrylamide, poly(N-vinyl pyrolidone), polyethylene oxide, and hydrolysed polyacrylonitrile. Examples of hydrogels which are organic polymers include covalent or ionically crosslinked polysaccharide-based hydrogels such as the polyvalent metal salts of alginate, pectin, carboxymethyl cellulose, heparin, hyaluronate and hydrogels from chitin, chitosan, pullulan, gellan and xanthan, The particular hydrogels used in our experiment were a cellulose compound (i.e. hydroxypropylmethylcellulose [HPMC]) and a high molecular weight hyaluronic acid (HA).

A drug delivery system within the scope of the present disclosure can be formulated with particles of an active agent dispersed within a biodegradable polymer. Without being bound by theory, it is believed that the release of the active agent can be achieved by erosion of the biodegradable polymer matrix and by diffusion of the particulate agent into an ocular fluid, e.g., the vitreous, with subsequent dissolution of the polymer matrix and release of the active agent. Factors which influence the release kinetics of active agent from the implant can include such characteristics as the size and shape of the implant, the size of the active agent particles, the solubility of the active agent, the ratio of active agent to polymer(s), the method of manufacture, the surface area exposed, the density of the implant and the erosion rate of the polymer(s).

The selection of the biodegradable polymer used can vary with the desired release kinetics, patient tolerance, the nature of the disease to be treated, and the like. Polymer characteristics that are considered include, but are not limited to, the biocompatibility and biodegradability at the site of implantation, compatibility with the active agent of interest, and processing temperatures. The biodegradable polymer matrix usually comprises at least about 10, at least about 20, at least about 30, at least about 40, at least about 50, at least about 60, at least about 70, at least about 80, or at least about 90 weight percent of the implant. In one variation, the biodegradable polymer matrix comprises about 40% to 50% by weight of the drug delivery system.

Biodegradable polymers which can be used include, but are not limited to, polymers made of monomers such as organic esters or ethers, which when degraded result in physiologically acceptable degradation products. Anhydrides, amides, orthoesters, or the like, by themselves or in combination with other monomers, may also be used. The polymers are generally condensation polymers. The polymers can be crosslinked or non-crosslinked.

Polylactide (PLA) polymers exist in 2 chemical forms, poly(L-lactide) and poly(D,L-lactide). The pure poly(L-lactide) is regioregular and therefore is also highly crystalline, therefore degrades in vivo at a very slow rate. The poly(D,L-lactide) is regiorandom which leads to more rapid degradation in vivo. Therefore a PLA polymer which is a mixture of predominantly poly(L-lactide) polymer, the remainder being a poly(D-lactide) polymer will degrade in vivo at a rate slower that a PLA polymer which is predominantly poly(D-lactide) polymer. A PLGA is a co-polymer that combines poly(D.L-lactide) with poly(glycolide) in various possible ratios. The higher the glycolide content in a PLGA the faster the polymer degradation.

The release rate of the active agent can depend at least in part on the rate of degradation of the polymer backbone component or components making up the biodegradable polymer matrix. For example, condensation polymers may be degraded by hydrolysis (among other mechanisms) and therefore any change in the composition of the implant that enhances water uptake by the implant will likely increase the rate of hydrolysis, thereby increasing the rate of polymer degradation and erosion, and thus increasing the rate of active agent release. The release rate of the active agent can also be influenced by the crystallinity of the active agent, the pH in the implant and the pH at interfaces.

The release kinetics of the drug delivery systems of the present invention can be dependent in part on the surface area of the drug delivery systems. A larger surface area exposes more polymer and active agent to ocular fluid, causing faster erosion of the polymer and dissolution of the active agent particles in the fluid.

The drug delivery systems may include a therapeutic agent mixed with or dispersed within a biodegradable polymer. The drug delivery systems compositions can vary according to the preferred drug release profile, the particular active agent used, the ocular condition being treated, and the medical history of the patient. Therapeutic agents which can be used in our drug delivery systems include, but are not limited to (either by itself in a drug delivery system within the scope of the present invention or in combination with another therapeutic agent): ace-inhibitors, endogenous cytokines, agents that influence basement membrane, agents that influence the growth of endothelial cells, adrenergic agonists or blockers, cholinergic agonists or blockers, aldose reductase inhibitors, analgesics, anesthetics, antiallergics, anti-inflammatory agents, antihypertensives, pressors, antibacterials, antivirals, antifungals, antiprotozoals, anti-infectives, antitumor agents, antimetabolites, antiangiogenic agents, tyrosine kinase inhibitors, antibiotics such as aminoglycosides such as gentamycin, kanamycin, neomycin, and vancomycin; amphenicols such as chloramphenicol; cephalosporins, such as cefazolin HCl; penicillins such as ampicillin, penicillin, carbenicillin, oxycillin, methicillin; lincosamides such as lincomycin; polypeptide antibiotics such as polymixin and bacitracin: tetracyclines such as tetracycline; quinolones such as ciproflaxin, etc.; sulfonamides such as chloramine T; and sulfones such as sulfanilic acid as the hydrophilic entity, anti-viral drugs, e.g. acyclovir, gancyclovir, vidarabine, azidothymidine, azathioprine, dideoxyinosine, dideoxycytosine, dexamethasone, ciproflaxin, water soluble antibiotics, such as acyclovir, gancyclovir, vidarabine, azidothymidine, dideoxyinosine, dideoxycytosine; epinephrine; isoflurphate; adriamycin; bleomycin; mitomycin; ara-C; actinomycin D; scopolamine; and the like, analgesics, such as codeine, morphine, keterolac, naproxen, etc., an anesthetic, e.g. lidocaine; β-adrenergic blocker or β-adrenergic agonist, e.g. ephidrine, epinephrine, etc.; aldose reductase inhibitor, e.g. epairestat, ponalrestat, sorbinil, tolrestat; antiallergic, e.g. cromolyn, beclomethasone, dexamethasone, and flunisolide; colchicine, anihelminthic agents, e.g. ivermectin and suramin sodium; antiamebic agents, e.g. chloroquine and chlortetracycline; and antifungal agents, e.g. amphotericin, etc., antiangiogenesis compounds such as anecortave acetate, retinoids such as Tazarotene, antiglaucoma agents, such as brimonidine (Alphagan and Alphagan P), acetozolamide, bimatoprost (Lumigan), timolol, mebefunolol; memantine, latanoprost (Xalatan); alpha-2 adrenergic receptor agonists; 2-methoxyestradiol; anti-neoplasties, such as vinblastine, vincristine, interferons; alpha, beta and gamma, antimetabolites, such as folic acid analogs, purine analogs, and pyrimidine analogs; immunosuppressants such as azathiprine, cyclosporine and mizoribine; miotic agents, such as carbachol, mydriatic agents such as atropine, protease inhibitors such as aprotinin, camostat, gabexate, vasodilators such as bradykinin, and various growth factors, such epidermal growth factor, basic fibroblast growth factor, nerve growth factors, carbonic anhydrase inhibitors, and the like.

Pharmaceutical compositions of an anti-complement antibody may be prepared for storage as a lyophilized formulation or aqueous solution by mixing the polypeptide having the desired degree of purity with optional pharmaceutically-acceptable carriers, excipients, or stabilizers typically employed in the art.

The composition should contain a sufficient amount of active ingredient to achieve the desired effect (referred to herein as the "effective amount" as can be readily determined by a person skilled in the art. In general, the solubility of the active ingredient in water and the concentration of the active ingredient needed in the tissue, guide the amount and rate of release of the agent. Compositions for systemic administration will require a different "effective amount" compared to compositions for direct injection into the eye or retina.

A person of ordinary skill in the art can easily determine an appropriate dosage to administer to a subject without undue experimentation. Typically, a physician will determine the actual dosage that will be most suitable for an individual subject based upon a variety of factors including the activity of the specific compound employed, the metabolic stability and length of action of the compound, the age, body weight, general health, diet, mode and time of administration, rate of excretion, drug combination, the severity of the particular condition, and the individual undergoing therapy. To determine a suitable dose, the physician or veterinarian could start doses levels lower than that required in order to achieve the desired therapeutic effect and gradually increase the dosage until the desired effect is achieved. Similarly, the number of administrations of the compositions described herein to achieve the desired effect may also be determined without undue experimentation. This is considered to be within the skill of the artisan and one can review the existing literature on a specific agent to determine optimal dosing.

In some embodiments, the composition is administered in the form of a liquid (e.g., drop or spray) or gel suspension. Alternatively, the composition is applied to the eye via liposomes or infused into the tear film via a pump-catheter system. Further embodiments embrace a continuous or selective-release device, for example, membranes such as, but not limited to, those employed in the OCUSERT System (Alza Corp., Palo Alto, Calif.) In an alternative embodiment, the p53 activator is contained within, carried by, or attached to a contact lens, which is placed on the eye. Still other embodiments embrace the use of the composition within a swab or sponge, which is applied to the ocular surface.

In some cases, the composition further comprises a pharmaceutically acceptable carrier, e.g., a pharmaceutically acceptable salt. Suitable ocular formulation excipients include FDA approved ophthalmic excipients, e.g., emulsions, solutions, solution drops, suspensions, and suspension drops. Other suitable classifications include gels, ointments, and inserts/implants.

Exemplary excipients for use in optimizing ocular formulations include alcohol, castor oil, glycerin, polyoxyl 35 castor oil, Tyloxapol, polyethylene glycol 8000 (PEG-8000), ethanol, glycerin, cremaphor, propylene glycol (pG), polypropylene glycol (ppG), and polysorbate 80. In some cases, citrate buffer and sodium hydroxide are included to adjust pH.

Preferably, the compositions are delivered by topical, intravitreal, intraocular, subretinal, or systemic administration. For example, the compositions are administered by intraocular injection or subretinal injection. The compositions may also be delivered systemically. Antibodies have been previously shown to be successfully administered and delivered via systemic delivery, such as anti-C5 antibody for the treatment of wet age-related macular degeneration (AMD). Although systemic administration of an anti-immune response therapeutic may have adverse side effects, such as increased occurrence of infections, there has been no evidence to date that shows that systemic administration of anti-C5 antibody caused sufficient suppression of the immune system to increase occurrence of infections.

As described in detail below, retinal detachment is accompanied by apoptosis in the retina. Specifically, photoreceptor cell death was detected in retinas after detachment. Furthermore, electroretinography was used to assess photoreceptor function after retinal detachment. The examples below demonstrate that statistically significant decreases in both α-wave and b-waves were detected by electroretinography after retinal detachment. Thus, photoreceptor function decreases as a result of the loss and/or death of photoreceptor cells.

Also as described in detail below, components of the alternative complement pathway have increased activity after retinal detachment. Increases in expression at both the RNA the protein level of alternative complement pathway components were detected after retinal detachment in both mouse models of RD and human patients suffering from RD. Therefore, the alternative complement pathway plays a critical role in retinal detachment.

As described in detail below, inactivation of components of the alternative complement pathway prevent photoreceptor cell death after retinal detachment. For example, inactivation of Fb or C3 was shown to reduce photoreceptor cell death. The effects of inactivation of alternative complement pathway components were also examined in mouse models of retinal detachment and human patients with retinal detachment.

### Example 1: Model of Retinal Detachment

Animal models of retinal detachment (RD) provide an understanding of the cellular mechanisms that facilitate photoreceptor cell death (Lewis G.P. et al., Eye (Lond), 2002). A mouse model of RD was utilized that provides a systematic and controlled system and allows the advantage of the genetic manipulation possible in mice (Matsumoto, H. et al., J Vis Exp, 2013, (79)). Briefly, RD was created in the right eye of adult mice (8-12 weeks), as previously reported (Hisatomi, T. et al., Am J Pathol, 2001, 158:1271-1278), with minor modification. Briefly, mice were anesthetized using a mixture of ketamine (80mg/kg) and xylazine (8mg/kg). Deep anesthesia was confirmed by a toe pinch test. One drop of proparacaine hydrochloride (0.5%) (Akorn, 17478-263-12) was administered to each eye. A self-sealing scleral incision was made using a 30G needle. Next, an anterior chamber puncture was performed from the cornea to reduce intraocular pressure. A 33G needle attached to a Hamilton 10µl syringe was inserted into the subretinal space, and 4µl sodium hyaluronate (Provisc®, Alcon) was gently injected to enlarge the RD (fundus Image of a retinal detachment; Fig. 7A) (Matsumoto, H. et al., J Vis Exp, 2013, (79)). After the injection, glue (Webglue™) was put on the scleral wound and the conjunctiva reattached to the original position. Photoreceptor apoptosis was assessed (12-72 hours post detachment) by identifying TUNEL positive cells in cross sections (Figs. 7C and D). Maximal photoreceptor cell death in response to RD was found to occur 24 hours post injury in this model (Fig. 7C). ERG analysis allowed for the non-invasive assessment of neural responses that can be used to objectively evaluate retinal function on a layer-by-layer basis following light stimulation (Weymouth A. E., et al., Prog Retin Eye Res, 2008, 27:1-44). RD results in a significant loss of retinal function (a- and b- wave) as demonstrated by ERG analysis. The levels of complement factor-B (FB) were assessed, a key component of the alternative pathway, in the vitreous of patients with and without (macular hole) RD.

### Example 2: Isolation of the ONL using laser capture micro-dissection

Eyes were enucleated 24 hours post RD then placed in OCT compound (Tissue Tek, 4583, Torrance, CA), and quickly frozen by submerging in a beaker of isopropanol chilled by dry ice. Between 16 and 18 sections cut at 30µm were placed onto a frame slide (leica 11505190, Germany) under RNAse free conditions then allowed to air dry for 10 minutes. All reagents were made using nuclease free water (Ambion, AM9932, Carlsbad, CA). The sections on the frame slides were fixed using a graded series of EtOH (Sigma, 459836-1L, St. Louis, MO) consisting of incubation in 50% EtOH for 1 minute, 75% EtOH for 1.5 minutes, and water for 1 minute. To identify the cell layers the slides were dipped in 0.1% toluidine blue solution (Fluka, 89640-5G, St. Louis, MO) followed by 2 rinses in water for 15 seconds each, 75% EtOH for 30 seconds, and water for 15 seconds. Sections were dried in room air then the photoreceptors were isolated using laser capture micro-dissection (Leica, LMD 7000, Germany). Samples were collected in RNAlater® solution (Ambion, AM7022, Carlsbad, CA) and stored at -80°C until RT-PCR was performed.

### Example 3: Alternative Complement Pathway in RD

Increased activity of the alternative complement pathway was correlated with retinal detachment. In this Example, gene expression and protein levels of alternative complement pathway components were assessed in the mouse model of RD.

To define the role of alternative complement pathway in RD a mouse model that allowed for the genetic manipulation possible in mice was utilized (Matsumoto, et al. J Vis Exp. 2013). In this model a sustained RD is created by a sub-retinal injection of sodium hylaluronate (Provisc®), resulting in approximately 60% of the retina becoming detached (Fig.1B) (Matsumoto, et al. J Vis Exp, 2013). Photoreceptor apoptosis is assessed from 12-72 hours post detachment by identifying TUNEL positive cells in the outer nuclear layer (ONL) (Figs. 7A-D). The peak amount of cell death occurs at 24 hours post-detachment (Figs. 7C and D). Fb expression in the retinas of mice with or without RD was assessed and found significant up-regulation from 12-48 hours after detachment, peaking at 24 hours (Figs. 1C and D). Key activators for the lectin (*Masp2*) and classical (*C1q*) complement pathways were also assessed and in both cases regulation was not as significant as the alternative pathway but there were some time points with minor, yet significant, changes (Fig. 8).

Cd55, a regulatory protein of the alternative pathway, is suppressed in the photoreceptors of RD mice. Soluble and cell bound regulators of complement help to protect healthy host tissue from self-recognition, providing protection from erroneous activation (Nesargikar, et al. Eur J Microbiol Immunol (Bp) 2, 103. Jun, 2012; Kemper et al. Clin Exp Immunol 124, 180. May, 2001; Harris, et al. Biochem J 341 (Pt 3), 821 Aug 1, 1999; Hamilton, et al. Blood 76, 2572. Dec 15, 1990). However, damaged or diseased host cells have been shown to down-regulate membrane bound regulators of complement, allowing for their targeted clearance (Suzuki et al. J Immunol 191, 4431. Oct 15, 2013; Banadakoppa et al. Cell Biol Int 36, 901. Oct 1, 2012; Gustafsson et al. Virology 405, 474. Sep 30, 2010. *Cd55,* a key regulator of the alternative pathway (Harris, et al. Biochem J 341 (Pt 3), 821 Aug 1, 1999), was found to be significantly down-regulated in the detached retina (Fig. 1C) making these cells intrinsically more prone to targeted cell death. To confirm that the decline in *Cd55* is specific to the photoreceptors the ONL was isolated using laser capture micro-dissection (Figs. 9A and B) and assessed gene expression by real-time PCR for *Cd55.*

RNA was isolated using an RNeasy micro kit (Qiagen, 74004, Valencia, CA) for LCM samples or an RNeasy mini kit (Qiagen, 74106, Valencia, CA) for whole retina. Total RNA was measured using a nanodrop spectrophotometer (Thermo Scientific, Nanodrop 2000, Waltham, MA) then each sample was normalized prior to transcribing cDNA. cDNA was transcribed using superscript III (Invitrogen, 18080-044, Carlsbad, CA) then one microliter of cDNA was used for each RTPCR reaction. Primers to Fb (Life Technologies, Mm004333909), C1q (Life Technologies, Mm00432142), MASP2 (Life Technologies, Mm00521963), Cd55 (Life Technologies Mm00438377), and Cd59a (Life Technologies, Mm00483149) were used for whole retina and combined with Taqman universal PCR master mix (Life tehcnologies, 4304437). Primers for Cd55 using photoreceptors isolated by LCM were obtained through Integrated DNA Technologies using their online Primer Quest design tool and importing the NCBI ID number for the sequence (Forward 5'-TGTAAGCAGAATCGCCACAGAGGT-3' (SEQ ID NO: 5) and Reverse 5'-GTGAGCTTCCACTGCAGGTTTGTT-3' (SEQ ID NO: 6)). RTPCR was run in triplicate and the average of each CT value used for analysis. All CT values were normalized to beta actin from each sample as an internal control. Final values were determined using the ΔΔCT method.

*Cd55* expression levels in the photoreceptors were significantly reduced (74.6%±5.262) in response to RD (Fig. 1E). In RD the photoreceptors appear to be highly susceptible to alternative pathway mediated cell death due, in part, to down-regulation of the complement regulator Cd55.

### Example 4: Alternative Complement Pathway in Human RD

In this Example, the role of the alternative complement pathway in retinal detachment was confirmed in human patients. Patient vitreous samples were collected and processed 1-14 days after diagnosis of symptoms or detachment. Control vitreous samples were obtained from patients with macular hole, which is a natural control for retinal detachment. Undiluted vitreous (0.3 to 1.0 mL volume) was obtained from patients during standard three-port pars plana vitrectomy under direct visual control by aspirating liquefied vitreous from the center of the vitreous cavity with a syringe before starting the infusion. Vitreous was obtained from 9 patients with varying degrees of retinal detachment (RD) and 4 patients that had a macular hole with no RD (control samples) (Fig. 5). Samples were kept on ice during the time of surgery then immediately moved to -80°C for storage. In order to separate the soluble protein from the collagenous matrix the samples were thawed on ice then spun at 12,000 RPM for 15 minutes at 4°C (J. Yu et al., Proteomics 8, 3667 (Sep, 2008). The supernatant was collected, aliquots taken, and stored at -80°C. Each aliquot was thawed not more than once for use in the ELISA assays.

Protein levels of factor-b (Fb), a key component in the alternative complement pathway, were assessed by ELISA. All human ELISA measurements were performed using 5µl of undiluted vitreous, isolated as described above. All ELISAs were performed following kit instructions and measured using a Molecular Devices Spectramax M3 plate reader. Standard curves were generated using the standard reagents provided in the kit then sample values determined automatically using Softmax pro software. Human ELISA kits that were used include Fb (Novatein, BG-HUM10501), Fd (Abcam, ab99969), C5 (Abcam, 125963), and C3 (Abcam, ab108822).

Factor-B was significantly up regulated in RD patients, indicating alternative pathway activation (Fig. 1A). Interestingly, there was no significant change in several other key complement proteins; including Factor D, C5, and C3 (Figs. 6C-E). This is in line with previous studies that have shown that there are key regulatory proteins that undergo transcriptional control to modulate the activity of the complement pathways (Hecker et al. Hum Mol Genet 19, 209. Jan 1, 2010; Nielsen, et al. Apmis 100, 1053. Dec, 1992; Suankratay, et al. Clin Exp Immunol 117, 442. Sep, 1999). For the alternative complement pathway FB has been described as a key effector molecule responsible for pathway activation (Hecker et al. Hum Mol Genet 19, 209. Jan 1, 2010). However inhibition of any component of the pathway is sufficient to block complement activation.

### Example 5: Complement Pathway Inactivation Prevents Photoreceptor Cell Death

The role of the alternative complement pathway was analyzed in photoreceptor cell death during retinal detachment. Transgenic knockout mice were utilized to assess the effect of alternative complement pathway inactivation on retinal cell death after retinal detachment.

Retinal detachment was induced in mice lacking alternative complement component Fb (Fb^{-/-}) and their wild-type (WT) littermates (age-matched control), as described in Example 1. Twenty-four hours post-injury, retinas were isolated and cross-sections were prepared for staining with DAPI (a nuclear marker) and TUNEL (a cell death marker). The mid-point of the retinal arc between the optic nerve and the edge of the retina was quantified for TUNEL positive cells. Representative staining of the retinal cross-sections are shown in Fig. 4A). Quantification of the cross sections (Fig. 3B) showed that mice lacking a functional alternative complement cascade were significantly protected from photoreceptor cell death. This data implicates the alternative complement system in initiating photoreceptor cell death. It also demonstrates that neutralizing or inhibiting the alternative complement cascade can be sufficient to protect from photoreceptor degeneration.

The experiment was also performed in mice lacking the complement component C3. Photoreceptor cell death was assessed 24 hours post-retinal detachment by TUNEL staining of retinal cross-sections. As shown in Fig. 2F, loss of the complement component-3 protects mice from photoreceptor cell death resulting from retinal detachment.

C3 neutralizing antibody: In order to dampen complement activation a neutralizing antibody was injected against the central C3 protein required for complement amplification. Prior to *in vivo* injection the azide was removed from the C3 antibody (Abcam, ab11862) using an Abcam purification kit (ab102784) following kit instructions. Briefly, 200µl of C3 antibody was used for starting material and incubated in in the provided resin containing 20µl of 10x binding buffer for 1 hour at room temperature with gentle agitation. The resin was then spun to remove unbound antibody and washed with the provided wash buffer. The antibody was eluted with 100µl of elution buffer then 25µl of neutralizer was added. The elution procedure was repeated 3 times using separate collecting tubes. Only the first tube was used for *in vivo* injections corresponding to 0.1mg/ml. The model of retinal detachment was performed precisely as described above with one modification: Prior to injecting the provisc 1µl of the purified C3 antibody (0.1µg) or IgG2a isotype control (Life Technologies, 02-9688) was injected into the sub-retinal space through the scleral tunnel. The eyes were enucleated 24 hours after RD and processed for TUNEL labeling as described earlier.

Fd neutralizing antibody: To dampen the alternative pathway an antibody was injected that binds to Fd (R&D systems, MAB5430) prior to creating RD. The antibody did not contain azide therefore was resuspended in PBS to a final concentration of 0.5mg/ml, aliquots taken, and stored in -20°C. The antibody was not thawed more than once for injection. The model of retinal detachment was performed precisely as described above with one modification: Prior to injecting the provisc® 1µl of the purified Fd antibody (0.5 µg) or IgG1 isotype control (Life Technologies, R100) was injected into the sub-retinal space through the scleral tunnel into the sub-retinal space. The eyes were enucleated 24 hours after RD and processed for TUNEL labeling as described earlier.

Conversely, restoration of the complement pathway ablates the protection provided by inactivation of the complement cascade. C3 knockout mice with retinal detachments were administered cobra venom factor (CVF) or PBS (control). Cobra venom factor is a protein that is a mimetic for normal endogenous C3/C3b and therefore restores the complement cascade in the C3 knockout mice. Mice without C3 and injected PBS were protected from photoreceptor cell death. However, mice injected with CVF and restoration of the complement cascade exhibited a significant up-regulation of photoreceptor cell death as a result of retinal detachment. This data demonstrates that the complement system facilitates photoreceptor cell death in response to injury (retinal detachment). Inhibition of this pathway protects photoreceptors from injury-mediated cell death and subsequent retinal degeneration.

Re-activation of the complement system in *C3*^{*-*/*-*} mice: To re-activate complement in *C3*^{*-*/*-*} mice 1µl (1.1mg) of cobra venom factor (CVF) (quidel, A600) into the sub-retinal space was injected prior to the Provisc® injection. When introduced into the blood stream CVF activates complement, bypassing the need for endogenous C3. The rest of the model of retinal detachment was performed precisely as described above. The eyes were enucleated 24 hours after RD and processed for TUNEL labeling as described earlier.

### Example 6: Alternative pathway deficient mice are protected from RD associated photoreceptor cell death

Photoreceptor cell death in response to RD was next assessed in a *C3*^{*-*/*-*} mouse, which lacks the central C3 protein required for all three complement pathways (Ricklin, et al. Nat Immunol 11, 785. Sep, 2010). Photoreceptor cell death was quantified at 24 hours post detachment, the peak of cell death. *C3*^{*-*/*-*} mice had a reduction in the number of TUNEL positive cells compared to their age and strain matched controls (Figs. 2A and B). To further define the role of C3 in RD, C57B16 (control wild type) mice were given injections of an antibody (Ab) against C3 in the sub-retinal space at the time of detachment. Quantitation of TUNEL labeling revealed that administration of an anti C3 Ab significantly protected the mice from photoreceptor cell death in response to RD (Figs. 2C and D). Conversely, complement was re-activated in *C3*^{*-*/*-*} mice by introducing cobra venom factor (CVF) which is a stable functional analog to C3b (Krishnan et al. Structure 17, 611. Apr 15, 2009). This bypasses the need for C3 by replacing the C3 cleavage product, C3b, required for the alternative pathway proteolytic cascade to continue. The reactivation of the complement system in *C3*^{*-*/*-*} mice using CVF increased photoreceptor cell death after RD (Figs. 2E and F). Taken together these results strongly implicate the complement system as a driving force in promoting the early photoreceptor cell death associated with RD.

The alternative complement pathway remains in a primed state through constant tickover of the central alternative pathway C3 protein allowing for continuous probing within the retinal microenvironment (and CNS) for the identification of cells that are damaged or dying; distinguishing the alternative pathway from the lectin and classical pathways (Ricklin, et al. Nat Immunol 11, 785. Sep, 2010; Bexborn, et al. Mol Immunol 45, 2370. Apr, 2008). To determine the role of the alternative pathway in photoreceptor cell death during RD mice that lack Fb, an essential rate limiting protein required for alternative pathway activation after C3 cleavage were tested (Hecker et al. Hum Mol Genet 19, 209. Jan 1, 2010). Mice deficient in the alternative complement pathway (*Fb*^{*-*/*-*}) had a substantial decrease in photoreceptor cell death 24 hours post RD (Figs. 3A and B). Complement factor-d (Fd) is a serine protease, which cleaves Fb once bound to C3b resulting in the assembly of the alternative pathways C3-convertase (Ricklin, et al. Nat Immunol 11, 785. Sep, 2010). To pharmacologically block the alternative complement pathway in RD an antibody against Fd was injected into the subretinal space of C57BL6 control mice at the time of detachment. Neutralization of Fd resulted in a reduction in the amount of photoreceptor cell death compared to isotype matched controls (Figs. 3C and D). Notably, mice deficient in either the lectin (*Mbl A*/*C*^{*-*/-}) or classical (*C1q*^{*-*/-}) pathways did not confer protection against complement mediated photoreceptor cell death (Figs. 10A-D). This data directly implicates the alternative complement pathway and not the lectin or classical in mediating photoreceptor cell death in response to RD.

### Example 7: Efficacy of Anti-Complement Agents as Therapeutics

Retinas are detached as described in Example 1. At the time of retinal detachment, the mice are administered either inhibitor or a saline control by intraocular injection, subretinal injection, or systemic administration. After 24 hours, retinal cross-sections are prepared and stained by TUNEL to assess photoreceptor cell death, as described in Example 5. Additional timepoints (*i.e.,* at 12 hours, 36 hours and 48 hours after retinal detachment) are assessed to verify the efficacy and optimal timing of administration.

Photoreceptor cell viability after treatment is assessed by electroretinography to determine photoreceptor cell death and extent of preservation of photoreceptors and vision. Retinal cross-sections are prepared, and stained with cell death markers (such as TUNEL) to determine increased or reduced photoreceptor cell death. Optomotor visual tests are performed to test preservation of vision.

### Example 8: Retinal hypoxia leads to alternative pathway activation and cell death in RD

Photoreceptor cells are one of the most highly metabolic cell types in the body (Linsenmeier, et al. Invest Ophthalmol Vis Sci 41, 3117. Sep, 2000). Yet with such high metabolic demand they are not permeated with a vascular network, deriving 90% of their nutrients and oxygen by diffusion from the vascular bed of the choroid/choriocapillaris (Linsenmeier, et al. Invest Ophthalmol Vis Sci 41, 3117. Sep, 2000; Luo, et al. Elife 2, e00324. 2013). When RD occurs it physically separates the photoreceptor cells from the RPE and distances them from the choroid thereby compromising access to nutrients and oxygen. Several seminal studies have shown that the deprivation of oxygen (hypoxia) is a leading cause of photoreceptor cell death (Lewis, et al. Mol Neurobiol 28, 159. Oct, 2003; Lewis, et al. Am J Ophthalmol 137, 1085. Jun, 2004) in RD. Studies were carried out to determine whether hypoxia could facilitate alternative complement pathway activation and photoreceptor cell death in response to RD.

To define global hypoxia in photoreceptors in response to RD mice were injected 22.5-hours after detachment intraperitoneally with a marker of hypoxia, Hypoxyprobe™. Hypoxyprobe™ is a thiol binding probe that only binds to cells with an oxygen concentration less than 14µmol which can be visualized through diaminobenzadine (DAB) amplification. In retinal cross sections the detached photoreceptors stained strongly, indicating hypoxia, whereas in the attached regions of the same cross section no staining could be observed (Fig. 4A).

To obtain a more precise reading of the retinal oxygen concentration *in vivo* a glass oxygen microsensor that contains a silicone membrane was used allowing for the diffusion of oxygen into an oxygen-reducing cathode. In vivo oxygen measurements were performed using a 50µm diameter glass-electrode-oxygen sensor (Unisense A/S, Denmark). This probe has a linear response between 0-1 atm pO₂ ranges, negligible oxygen consumption (10-16 mol/sec) and fast time response. The cathode is polarized against an internal Ag/AgCl anode that results in rapid, 0.3 second, *in vivo* oxygen measurements. Prior to its use, the sensor was thoroughly pre-polarized for 24 hours and was calibrated using two points: maximum oxygen saturation using 0.9% saline at 37° C under continuous air agitation, and zero oxygen calibration using sodium ascorbate solution. A linear fit was calculated from the two calibration points and implemented into the data acquisition software. Sampling rate was adjusted to 1Hz (Nyquist-Shannon sampling theorem) and measurements were acquired and stored in a portable computer for later processing.

Placing the probe into the attached retina using a stereotaxic frame it was defined an average oxygen concentration of 47.64±3.346µmol compared to the contralateral, detached, retina with an average oxygen concentration of 17.14±5.031µmol (Fig. 4B). To assess if the hypoxic state of the photoreceptors facilitates alternative pathway mediated cell death RD in mice housed in room air (approximately 21% oxygen) or housed in a chamber maintained at 75% oxygen were analyzed. After 24 hours, eyes were enucleated and TUNEL labeling performed. Quantitation of TUNEL positive cells in the ONL revealed a significant reduction in cell death within the group of mice maintained at 75% oxygen (Figs. 4C and D). Importantly, the mice kept in 75% oxygen had significantly less Fb expression, alternative pathway activation, than their room air counterparts (Fig. 4E) indicating an oxygen dependent regulation of Fb.

Mice were anesthetized by using a mixture of ketamine (80mg/kg) and xylazine (8mg/kg) administered via an intra-peritoneal (IP) injection. Adequate sedation was confirmed by toe pinch. One drop of proparacaine hydrochloride (0.5%) (Akorn, 17478-263-12) was administered to each eye. A stereotaxic frame (Leica 39463001) with Cunningham mouse adaptor (Leica 39462950) was used to immobilize the head of the mouse. A 32 gauge needle was then used to create a tunnel into the subretinal space. A manual (x,y,z) microstage, adapted with the oxygen sensor, was used to gently guide the probe into the subretinal space adjacent to the detached retina and measurements were performed for several seconds, until oxygen readings were stabilized. Oxygen measurements in the contralateral non-detached eyes served as internal controls and naive mice served as control reference. During the experimental time frame the probe was retested for accuracy in the calibration solutions and found to be within range. The mice were euthanized by spinal dislocation after the final reading.

### Example 9: Detecting hypoxic regions in the retina

Hypoxic areas of the retina were identified 24 hours after detachment with the use of the Hypoxyprobe™-1 Plus kit (HPI, HP2-100). Hypoxyprobe™ (pimonidazole hydrochloride) is a substituted 2-nitoimidazole that forms adducts with thiol containing proteins in only those cells that have an oxygen concentration less than 14µmol (e.g. hypoxic cells). Retinal detachments were made as described above and 22.5 hours later mice were anesthetized with a mixture of ketamine (80mg/kg) and xylazine (6mg/kg) then injected with Hypoxyprobe™ (2mg in a volume of 100µl) directly into the left ventricle. Mice were kept on a heating pad maintained at 37°C and monitored for 90 minutes to allow complete binding of the probe to hypoxic tissues. The eyes were enucleated and frozen in OCT that had been chilled in dry ice. Blocks were sectioned at 20µm then fixed in 4%PFA for 20 minutes. The endogenous peroxidase present in the tissue was quenched using 3% H₂O₂ for 5 minutes. Non-specific binding was blocked using 5% fetal calf serum/0.5% triton x/0.3% bovine serum albumin. Samples were then incubated with a FITC conjugated mouse IgG1 that binds to pimonidizole (Hypoxyprobe™) at a dilution of 1:100. The FITC was amplified using rabbit anti-FITC conjugated with horseradish peroxidase at a dilution of 1:75 followed by the addition of a brown DAB chromagen. To identify the retinal layers slides were dipped in haematoxylin (Sigma, H9627) for 1 minute then washed 3 times in PBS and coverslipped. Binding of the probe was compared on retinal cross sections where 50% of the retina was attached and the other 50% detached.

### Example 10: Supplementing oxygen after retinal detachment

Retinal detachments were performed as described above then the mice were kept either in the animal holding facility (≈21% O₂) or immediately placed into an oxygen chamber maintained at constant 75% O₂ (Biospherix model 110). After 24 hours the mice were anesthetized under a heavy dose of avertin (2,2,2 tribromo ethanol, Sigma, T4, 840-2) at a working concentration of 10mg/ml and injected at a dose of 0.25-0.5 mg/gram intraperitoneally. In one group the eyes were enucleated and frozen in OCT compound using isopropanol chilled by dry ice. The eyes were then processed for TUNEL labeling and quantified as described above. In the other group the retina was removed and immediately flash frozen in liquid nitrogen for RNA extraction. All eyes were stored at -80°C.

### Example 11: Clinical Trial for Anti-Complement Therapeutics for Treating RD

A clinical trial is performed to determine the efficacy of anti-complement therapeutics in human patients suffering from retinal detachment. Preferably, the anti-complement inhibitors are administered to subjects as soon as possible once the retinal detachment was evident. The patients are diagnosed with retinal detachment using fundus imagery, ophthalmoscope, or ultrasonography. A clinical professional or physician may also diagnose retinal detachment by identifying at least one symptom associated with retinal detachment. Other patient cohorts, such as those suffering from various ocular disorders with an increased risk of retinal detachment are also assessed.

Anti-complement inhibitors are administered to the subjects systemically, by intraocular injection or sub-retinal injection. The inhibitors are administered within 12 hours, 24 hours, or 48 hours of the diagnosis of retinal detachment. Placebos are administered via the same delivery methods as a control.

Assessment of the efficacy of treatment is determined in the subjects before and after delivery of the anti-complement inhibitor or the placebo. Efficacy of treatment is determined by evaluating photoreceptor cell activity and vision. Electroretinography is used to measure photoreceptor cell activity. Electrodes are placed on the cornea and/or on the skin surrounding the eye. During recording, the patient's eyes are exposed to standardized light stimulus (flash or pattern stimulus), and the resulting signal is displayed and analyzed.

Spectral domain optical coherence tomography (SD-OCT) is also used for detailed and non-invasive evaluation of the retinal architecture in vivo. SD-OCT is used to show retinal morphological changes during retinal detachment.

Standard eye examinations are also performed to evaluate vision preservation or loss. Visual acuity is tested using Sneller charts, to test the ability of the subject to distinguish varying sizes of letters and/or numbers from a distance. Peripheral vision can also be tested using visual field exams.

## Claims

1. A composition for use in the treatment or alleviation of a symptom of retinal detachment in a subject, wherein said composition comprises an agent that inhibits or reduces alternative complement pathway activity, wherein said agent comprises an anti-Factor D antibody or an antigen-binding fragment thereof.

2. A composition for use in the inhibition or reduction of photoreceptor cell death in a subject, wherein said composition comprises an agent that inhibits or reduces alternative complement pathway activity, wherein said agent comprises an anti-Factor D antibody or an antigen-binding fragment thereof

3. The composition for use according to claim 1 or 2, wherein said composition is suitable for intraocular injection or systemic administration.

4. The composition for use according to any one of the preceding claims, wherein the subject suffers from retinal detachment.

5. The composition for use according to any one of the preceding claims, wherein the subject suffers from trauma-induced retinal detachment.

6. The composition for use according to any one of the preceding claims, wherein said composition further comprises a pharmaceutically acceptable carrier, or wherein said composition is suitable for topical, intraocular, intravitreal, subretinal, or systemic administration.

7. The composition for use according to claim 2, wherein the subject suffers from retinal detachment, wherein the retinal detachment is induced by trauma, or wherein said composition is administered within 1 minute, 5 minutes, 10 minutes, 15 minutes, 30 minutes, 45 minutes, 1 hour, 2 hours, 3 hours, 4 hours, 5 hours, 6 hours, 7 hours, 8 hours, 9 hours, 10 hours, 12 hours, 14 hours, 16 hours, 18 hours, 20 hours, 22 hours, or 24 hours after retinal detachment.

8. The composition for use according to claim 1, wherein the retinal detachment is induced by trauma, wherein said symptom is selected from observing floaters in the field of vision, observing flashes of light, photoreceptor cell death, loss in peripheral vision, central vision loss, decrease in visual acuity, and blindness or wherein said composition is administered within 1 minute, 5 minutes, 10 minutes, 15 minutes, 30 minutes, 45 minutes, 1 hour, 2 hours, 3 hours, 4 hours, 5 hours, 6 hours, 7 hours, 8 hours, 9 hours, 10 hours, 12 hours, 14 hours, 16 hours, 18 hours, 20 hours, 22 hours, or 24 hours after retinal detachment.

9. A composition for use according to any preceding claims, wherein said composition is administered by intraocular injection.

## Patentansprüche

1. Zusammensetzung zur Verwendung bei der Behandlung oder Linderung eines Symptoms einer Netzhautablösung in einem Subjekt, wobei die Zusammensetzung ein Mittel umfasst, das die Aktivität des alternativen Komplementwegs inhibiert oder verringert, wobei das Mittel einen Anti-Faktor-D-Antikörper oder ein Antigen-bindendes Fragment davon umfasst.

2. Zusammensetzung zur Verwendung bei der Inhibition oder Verringerung von Photorezeptor-Zelltod in einem Subjekt, wobei die Zusammensetzung ein Mittel umfasst, das die Aktivität des alternativen Komplementwegs inhibiert oder verringert, wobei das Mittel einen Anti-Faktor-D-Antikörper oder ein Antigen-bindendes Fragment davon umfasst

3. Zusammensetzung zur Verwendung nach Anspruch 1 oder 2, wobei die Zusammensetzung zur intraokularen Injektion oder systemischen Verabreichung geeignet ist.

4. Zusammensetzung zur Verwendung nach einem beliebigen der vorangehenden Ansprüche, wobei das Subjekt an Netzhautablösung leidet.

5. Zusammensetzung zur Verwendung nach einem beliebigen der vorangehenden Ansprüche, wobei das Subjekt an Trauma-induzierter Netzhautablösung leidet.

6. Zusammensetzung zur Verwendung nach einem beliebigen der vorangehenden Ansprüche, wobei die Zusammensetzung weiterhin einen pharmazeutisch annehmbaren Träger umfasst, oder wobei die Zusammensetzung zur topischen, intraokularen, intravitrealen, subretinalen oder systemischen Verabreichung geeignet ist.

7. Zusammensetzung zur Verwendung nach Anspruch 2, wobei das Subjekt an Netzhautablösung leidet, wobei die Netzhautablösung durch Trauma induziert ist/ wird, oder wobei die Zusammensetzung innerhalb von 1 Minute, 5 Minuten, 10 Minuten, 15 Minuten, 30 Minuten, 45 Minuten, 1 Stunde, 2 Stunden, 3 Stunden, 4 Stunden, 5 Stunden, 6 Stunden, 7 Stunden, 8 Stunden, 9 Stunden, 10 Stunden, 12 Stunden, 14 Stunden, 16 Stunden, 18 Stunden, 20 Stunden, 22 Stunden oder 24 Stunden nach Netzhautablösung verabreicht wird.

8. Zusammensetzung zur Verwendung nach Anspruch 1, wobei Netzhautablösung durch Trauma induziert ist/ wird, wobei das Symptom ausgewählt ist aus Beobachten von Schwebepunkten im Gesichtsfeld, Beobachten von Lichtblitzen, Tod von Photorezeptorzellen, Verlust des peripheren Sehens, Verlust des zentralen Sehens, Abnahme der Sehschärfe, und Blindheit, oder wobei die Zusammensetzung innerhalb von 1 Minute, 5 Minuten, 10 Minuten, 15 Minuten, 30 Minuten, 45 Minuten, 1 Stunde, 2 Stunden, 3 Stunden, 4 Stunden, 5 Stunden, 6 Stunden, 7 Stunden, 8 Stunden, 9 Stunden, 10 Stunden, 12 Stunden, 14 Stunden, 16 Stunden, 18 Stunden, 20 Stunden, 22 Stunden oder 24 Stunden nach Netzhautablösung verabreicht wird.

9. Zusammensetzung zur Verwendung nach einem beliebigen der vorangehenden Ansprüche, wobei die Zusammensetzung mittels intraokularer Injektion verabreicht wird.

## Revendications

1. Composition pour une utilisation dans le traitement ou l'atténuation d'un symptôme de décollement rétinien chez un sujet, dans laquelle ladite composition comporte un agent qui inhibe ou réduit l'activité de la voie alterne d'activation du complément, dans laquelle ledit agent comporte un anticorps anti-Facteur D ou un fragment de liaison à l'antigène de celui-ci.

2. Composition pour une utilisation dans l'inhibition ou la réduction de la mort cellulaire des photorécepteurs chez un sujet, dans laquelle ladite composition comporte un agent qui inhibe ou réduit l'activité de la voie alterne d'activation du complément, dans laquelle ledit agent comporte un anticorps anti-Facteur D ou un fragment de liaison à l'antigène de celui-ci.

3. Composition pour une utilisation selon la revendication 1 ou 2, dans laquelle ladite composition est adaptée pour une injection intraoculaire ou une administration systémique.

4. Composition pour une utilisation selon l'une quelconque des revendications précédentes, dans laquelle le sujet souffre d'un décollement rétinien.

5. Composition pour une utilisation selon l'une quelconque des revendications précédentes, dans laquelle le sujet souffre d'un décollement rétinien induit par un traumatisme.

6. Composition pour une utilisation selon l'une quelconque des revendications précédentes, dans laquelle ladite composition comporte en outre un vecteur pharmaceutiquement acceptable, ou dans laquelle ladite composition est adaptée pour une administration topique, intraoculaire, intravitréenne, sous-rétinienne ou systémique.

7. Composition pour une utilisation selon la revendication 2, dans laquelle le sujet souffre d'un décollement rétinien, dans laquelle le décollement rétinien est induit par un traumatisme, ou dans laquelle ladite composition est administrée moins de 1 minute, 5 minutes, 10 minutes, 15 minutes, 30 minutes, 45 minutes, 1 heure, 2 heures, 3 heures, 4 heures, 5 heures, 6 heures, 7 heures, 8 heures, 9 heures, 10 heures, 12 heures, 14 heures, 16 heures, 18 heures, 20 heures, 22 heures ou 24 heures après le décollement rétinien.

8. Composition pour une utilisation selon la revendication 1, dans laquelle le décollement rétinien est induit par un traumatisme, dans laquelle ledit symptôme est choisi parmi l'observation de corps flottants dans le champ de vision, l'observation de flashes de lumière, la mort cellulaire de photorécepteurs, la perte de la vision périphérique, la perte de la vision centrale, la baisse de l'acuité visuelle, et la cécité, ou dans laquelle ladite composition est administrée moins de 1 minute, 5 minutes, 10 minutes, 15 minutes, 30 minutes, 45 minutes, 1 heure, 2 heures, 3 heures, 4 heures, 5 heures, 6 heures, 7 heures, 8 heures, 9 heures, 10 heures, 12 heures, 14 heures, 16 heures, 18 heures, 20 heures, 22 heures ou 24 heures après le décollement rétinien.

9. Composition pour une utilisation selon l'une quelconque des revendications précédentes, dans laquelle ladite composition est administrée par injection intraoculaire.
